# EUROPEAN PATENT SPECIFICATION

(11) **EP 1 772 095 B1**
(45) Date of publication and mention of the grant of the patent: **26.01.2011**
(21) Application number: 05761727.6
(22) Date of filing: 25.07.2005
(51) Int. Cl.: A61B 1/00, A61B 17/32

(54) **ENDOSCOPE**
ENDOSKOP
ENDOSCOPE

(30) Priority: 23.07.2004 JP 2004216355; 23.07.2004 JP 2004216356
(43) Date of publication of application: 11.04.2007
(73) Proprietor: Olympus Corporation, Tokyo 151-0072 (JP)
(72) Inventor: YAMAYA, Koji, Kuboyama-cho Hachioji-shi TOKYO 1928512 (JP)
(74) Representative: von Hellfeld, Axel
(86) International application number: PCT/JP2005/013571
(87) International publication number: WO 2006/009280

(56) References cited:
- EP-A1- 1 426 005
- JP-A- 2003 310 545
- JP-A- 2004 173 963

## Description

### Technical Field

The present invention relates to an endoscope including an instrument swing table provided at a distal end of an endoscope inserting portion configured to swinging an instrument inserted into an instrument insertion channel provided to the inserting portion and a swing table mechanism configured to remotely operate the instrument swing table from an operating portion.

### Background Art

In recent years, it is widely recognized that a technique of removing an affected region in a body cavity while observing the affected region with an endoscope is useful. The endoscope used for the technique of removing an affected region in endoscope observation is proposed by US Patent No. 6,458,074 and Japanese Patent Laid-Open No. 2003-204930 for instance.

The endoscope proposed by US Patent No. 6,458,074 is provided with a first forceps raising mechanism configured to raise an instrument in a first direction at a distal end opening of a first instrument insertion channel opened at a distal end of an inserting portion and a second forceps raising mechanism configured to raise an instrument in a second direction different from the first forceps raising mechanism at the distal end opening of a second instrument insertion channel opened at the distal end of the inserting portion. The raising directions of the two forceps raising mechanisms are different directions in at least two combinations. To be more specific, the endoscope allows the instrument which is the forceps used for the technique of removing an affected region to be projected, raised or swung in two different directions from the distal end of the inserting portion under observation by the endoscope.

The endoscope proposed by Japanese Patent Laid-Open No. 2003-204930 includes an instrument swing table operating mechanism configured to remotely operate an instrument swing table which is the forceps raising mechanism configured to control the raising direction of the instrument provided at the distal end opening of the instrument insertion channel opened at the distal end of the inserting portion proposed by US Patent No. 6,458,074 in both directions by using two operating wires. The instrument swing table operating mechanism can remotely operate and control the projection, raising or swinging directions of the instrument projected from the distal end of the inserting portion of the endoscope according to the above-mentioned US Patent No. 6,458,074 by using two operating wires.

Thus, an instrument swing table operating function is the function of raising or swinging the instrument projected from the distal end of the inserting portion in a predetermined direction, where it pulls the two operating wires provided on the instrument swing table for the sake of remotely operating rasing and swinging of the instrument swing table.

As for the structure of pulling the operating wires, a rotating operation of a swing table operating knob provided on a user's side of the operating wires is performed (rotational motion), and the rotational movement of the swing table operating knob is converted to linear motion so as to pull the operating wires.

Even when the rotating operation of the swing table operating knob is performed at an even speed, however, swinging speed in both directions of the instrument swing table provided at the distal end of the inserting portion was not even. As a rotational position of the instrument swing table at which the swinging of the instrument is fastest is not prescribed in a swinging range of the instrument with the instrument swing table, it becomes different according to the endoscope.

For this reason, in the case of using incision instruments such as a needle-like electric knife and a high-frequency knife on the instrument swing table in particular, swinging speed of the instruments is different depending on which part in an observation field of the endoscope the instruments are swung in even if the swing table operating knob on the user's side is operated to rotate at an even rotating operation speed.

In general, an image of the affected region is picked up at the center of a screen of an endoscope image in the observation and curative treatment of the affected region with the endoscope. For this reason, the affected region located in a peripheral part of the screen of the endoscope is hardly seeable because it is not a front view, and the image looks distorted due to influence of an aberration or the like of an observation optical system. Thus, in the case of incising a mucous membrane of a peripheral part of the affected region with the incision instrument, it is necessary to pay attention to a swing table operation of the incision instrument, that is, the swinging speed in particular, which is a fatigue factor of an operator.

As described in Japanese Patent Laid-Open No. 2003-305001 for instance, there is a known endoscope, as regards mucous membrane incision, which washes away mucus sticking to the mucous membrane and bleed on incision by means of water delivered from a front supply port so as to facilitate the observation of the mucous membrane as a subject of the incision from an observation window.

In the case of the conventional technique of incising a submucosa with a high-frequency knife inserted into the instrument insertion channel of the endoscope including the instrument swing table, a tissue of the incised mucous membrane sticks to a blade edge of the high-frequency knife when incising the submucosa by repeatedly swinging the high-frequency knife. There was a problem that the tissue of the mucous membrane sticking to the blade edge of the high-frequency knife carbonizes soon to blunt the high-frequency knife.

For this reason, once the tissue of the mucous membrane sticks to the blade edge of the high-frequency knife, the high-frequency knife had to be pulled out of the endoscope once amid performance of the incision technique, the tissue of the mucous membrane sticking to the high-frequency knife had to be cleanly removed, and then the high-frequency knife had to be inserted into the endoscope again to continue the incision of the mucous membrane. This had to be repeated many times in one case, and so an incision process was very inefficient.

EP 1 426 005 A1 discloses that an edge portion of an endoscope inserting portion includes an observation optical system, a first treatment-tool oscillating base which guides, in a first direction, a first treatment-tool guided via a first channel for inserting the treatment tool, and a second treatment-tool oscillating base which guides, in a second direction, a second treatment tool guided via a second channel for inserting the treatment tool.

The present invention has been made in view of the circumstances, and an object thereof is to provide an endoscope which excels in operability of the curative treatment performed by swinging the instrument projected from the instrument swing table at the distal end of the inserting portion in the endoscope observation and allows the fatigue caused to the operator to be alleviated.

Furthermore, it is possible, according to the present invention, to realize the endoscope of high efficiency in the incision process which can effectively remove the tissue of the mucous membrane sticking to the high-frequency knife and continue the incision process of the mucous membrane without pulling the high-frequency knife out of the instrument insertion channel in the incision of the mucous membrane with the high-frequency knife projected from the instrument swing table at the distal end of the inserting portion of the endoscope.

### Disclosure of the Invention

### Means for Solving the problem

A first endoscope of the present invention is the one comprising: observation means provided at a distal end of an endoscope inserting portion; an instrument insertion channel provided to the endoscope inserting portion; an instrument swing table configured to swing an instrument inserted into the instrument insertion channel and led out of the distal end of the endoscope inserting portion; a swing table operating portion configured to remotely operate the instrument swing table on a user's side; and a control mechanism configured to control swinging of the instrument swing table so that, when operating the swing table operating portion at a constant speed, a swinging speed in the case where the distal end of the instrument led out of the distal end of the endoscope inserting portion is at a center of a screen of an endoscope image obtained by the observation means is faster than the swinging speed in the case where the distal end of the instrument is in a peripheral part of the screen of the endoscope image.

A second endoscope is the one comprising: an instrument insertion channel provided to the endoscope inserting portion; an observation optical system including an observation window provided at a distal end of an endoscope inserting portion; an instrument swing table configured to swing an instrument led out of the distal end via the instrument insertion channel in an approximately horizontal direction or an approximately vertical direction of a screen of an endoscope image picked up by the observation optical system; and an instrument swing table operating portion configured to remotely operate the instrument swing table on a user's side, wherein, if the swing table operating portion is operated at a constant speed, the distal end of the instrument led out of the distal end moves faster in the case where the distal end of the instrument is at a center of the endoscope image than in the case where the distal end of the instrument is in a peripheral part of the endoscope image.

### Brief Description of the Drawings

Fig. 1 is a perspective view showing an overall configuration of an endoscope according to a first embodiment of the present invention;
Fig. 2 is a plan view showing a configuration of a distal end of an endoscope inserting portion of the endoscope according to the first embodiment of the present invention;
Fig. 3 is a perspective view showing the distal end of the endoscope inserting portion according to the first embodiment of the present invention;
Fig. 4 is a plan view showing a distal end component except a front face of an electric insulating cover of the distal end of the endoscope inserting portion according to the first embodiment of the present invention;
Fig. 5 is a perspective view showing the configuration of a first instrument swing table provided at the distal end of the endoscope inserting portion according to the first embodiment of the present invention;
Fig. 6 is a sectional view showing a cross-section cut at a VI to VI line of Fig. 2 of the distal end of the endoscope inserting portion for the sake of describing operation according to the first embodiment of the present invention;
Fig. 7 is a sectional view showing a relation between a first instrument swing table and an instrument provided at the distal end of the endoscope inserting portion, which is cut at a VII to VII line of Fig. 2 according to the first embodiment of the present invention;
Fig. 8 is a sectional view showing the cross-section of the endoscope inserting portion for the sake of describing the operation as with Fig. 7 according to the first embodiment of the present invention;
Fig. 9 is a sectional view showing an internal configuration of a first swing table operating portion
provided to an endoscope operating portion according to the first embodiment of the present invention;
Fig. 10 is a plan view viewed from an F direction of Fig. 9 and showing the internal configuration of the first swing table operating portion provided to the endoscope operating portion according to the first embodiment of the present invention;
Fig. 11 is a plan view viewed from a G direction of Fig. 9 and showing the internal configuration of the first swing table operating portion provided to the endoscope operating portion according to the first embodiment of the present invention;
Fig. 12 is a plan view showing a state of having rotationally driven from the state of Fig. 11 and shows the internal configuration of the first swing table operating portion provided to the endoscope operating portion according to the first embodiment of the present invention;
Fig. 13 is an explanatory diagram for describing an operational state of the endoscope according to the first embodiment of the present invention;
Fig. 14 is an explanatory diagram for describing a swinging state of the instrument in an endoscope image of the endoscope according to the first embodiment of the present invention;
Fig. 15 is an explanatory diagram for describing a swinging operation of the instrument in the endoscope image according to the first embodiment of the present invention;
Fig. 16 is an explanatory diagram for describing a cleaning state by the endoscope inserting portion in the endoscope image according to the first embodiment of the present invention;
Fig. 17 is a sectional view showing the cleaning state of the distal end of the endoscope inserting portion according to the first embodiment of the present invention;
Fig. 18 is a sectional view showing the configuration of the first instrument swing table operating portion of the endoscope according to a second embodiment of the present invention;
Fig. 19 is an explanatory diagram for describing an action on an endoscope image screen of the instrument operated by the first instrument swing table operating portion of the endoscope according to the second embodiment of the present invention;
Fig. 20 is a plan view showing the configuration of a distal end face of the distal end of the endoscope inserting portion according to a third embodiment of the present invention;
Fig. 21 is a sectional view cut at a section line XXI to XXI of Fig. 20 and showing the configuration of the distal end of the endoscope inserting portion according to the third embodiment of the present invention;
Fig. 22 is an explanatory diagram for describing the screen of the endoscope image observed by the endoscope according to the third embodiment of the present invention;
Fig. 23 is a plan view showing the configuration of the distal end of the endoscope according to a fourth embodiment of the present invention;
Fig. 24 is a sectional view showing the configuration of the distal end of the endoscope, which is cut at a section line XXIV to XXIV shown in Fig. 23 according to the fourth embodiment of the present invention;
Fig. 25 is a sectional view showing the configuration of the distal end of the endoscope, which is cut at a section line XXV to XXV shown in Fig. 23 according to the fourth embodiment of the present invention;
Fig. 26 is a sectional view for describing a detergent action of the distal end of the endoscope according to the fourth embodiment of the present invention;
Fig. 27 is a plan view showing the configuration of the distal end of the endoscope according to a fifth embodiment of the present invention;
Fig. 28 is an explanatory diagram for describing the swinging operation of the instrument in the endoscope image of the endoscope according to the fifth embodiment of the present invention; and
Fig. 29 is an explanatory diagram for describing an operation of the endoscope according to the fifth embodiment of the present invention.

### Best Mode for Carrying Out the Invention

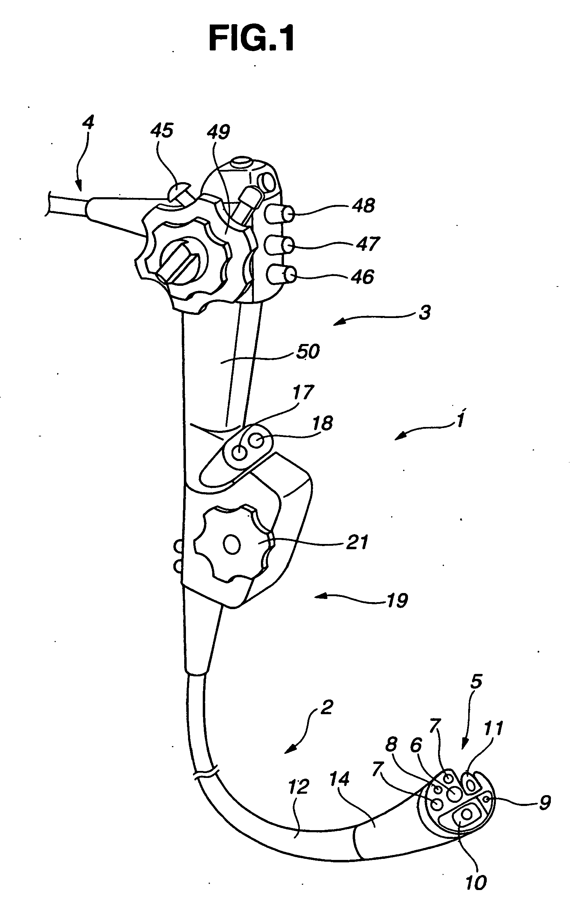

Hereunder, embodiments of the present invention will be described in detail with reference to the drawings. A first embodiment of an endoscope of the present invention will be described by using Figs. 1 to 16.

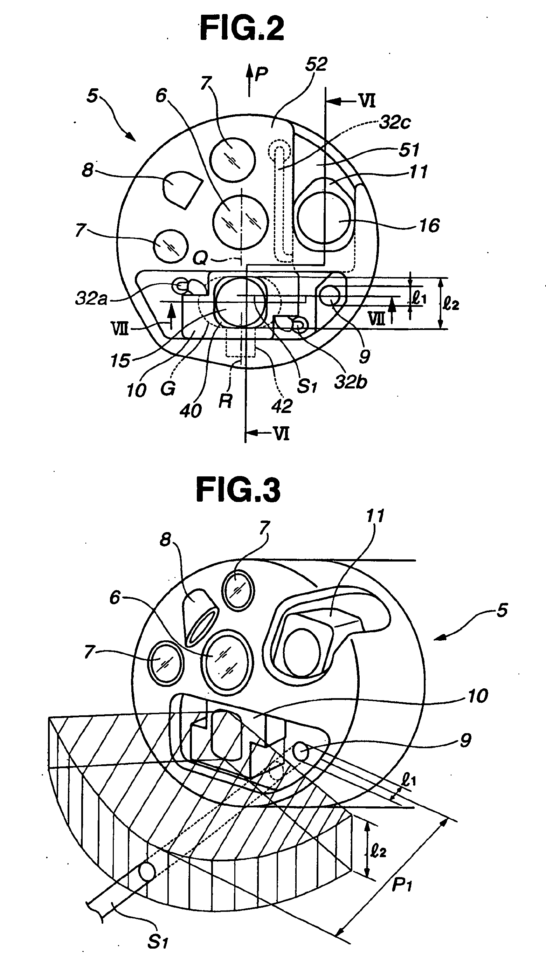

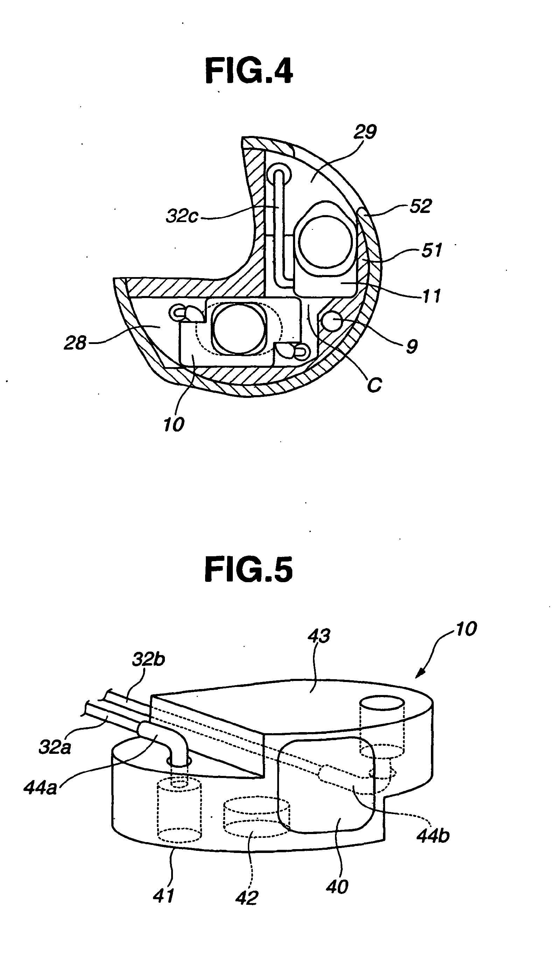

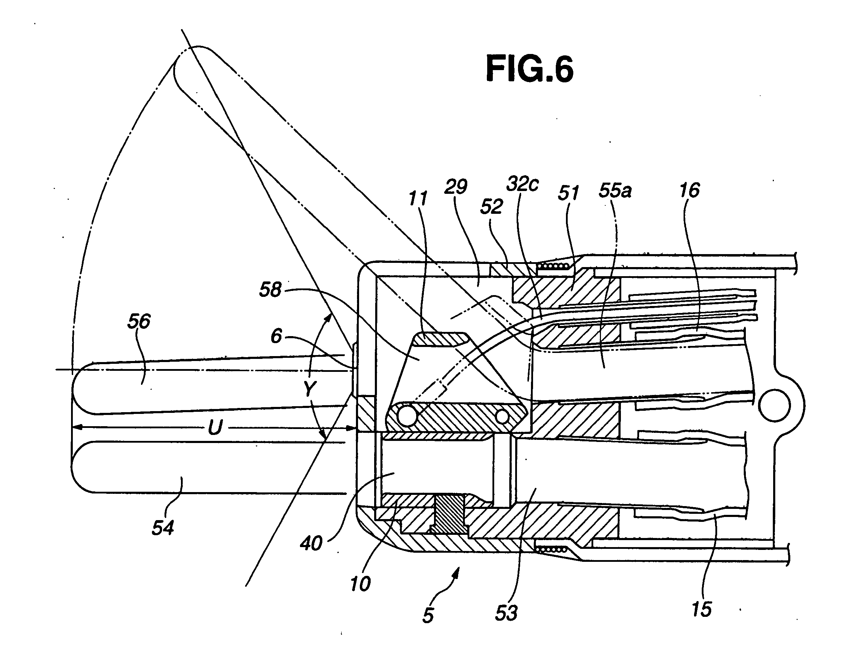

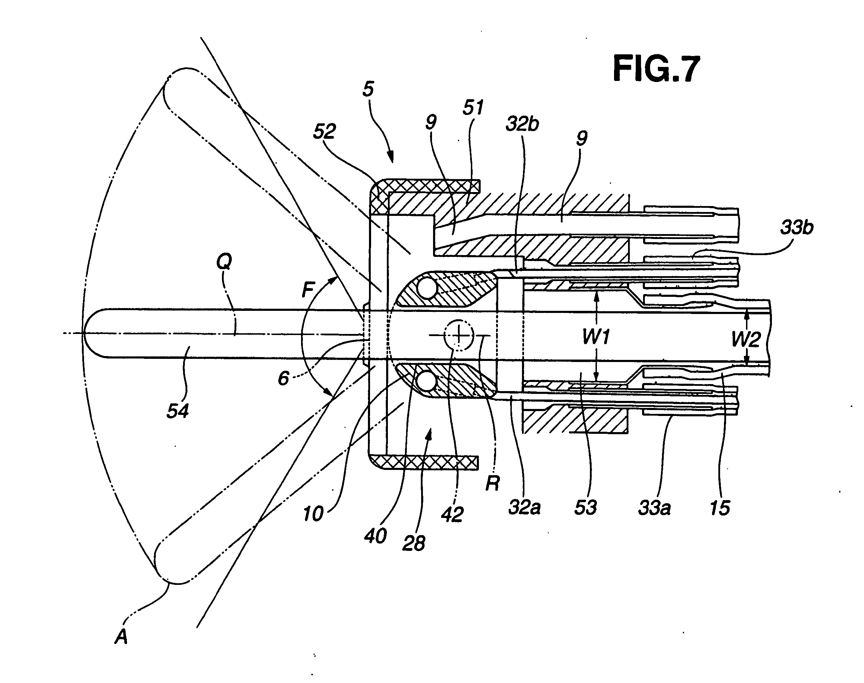

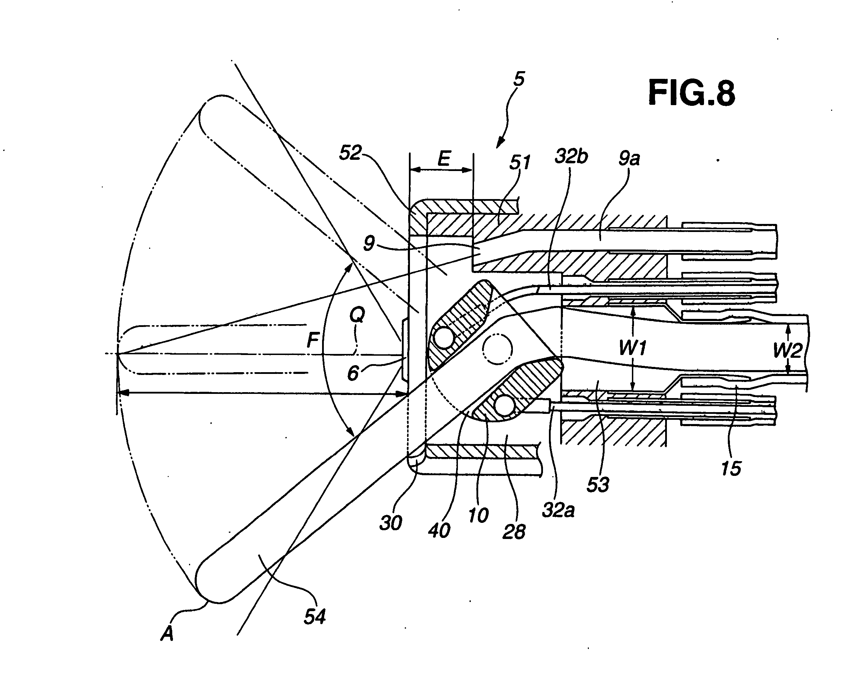

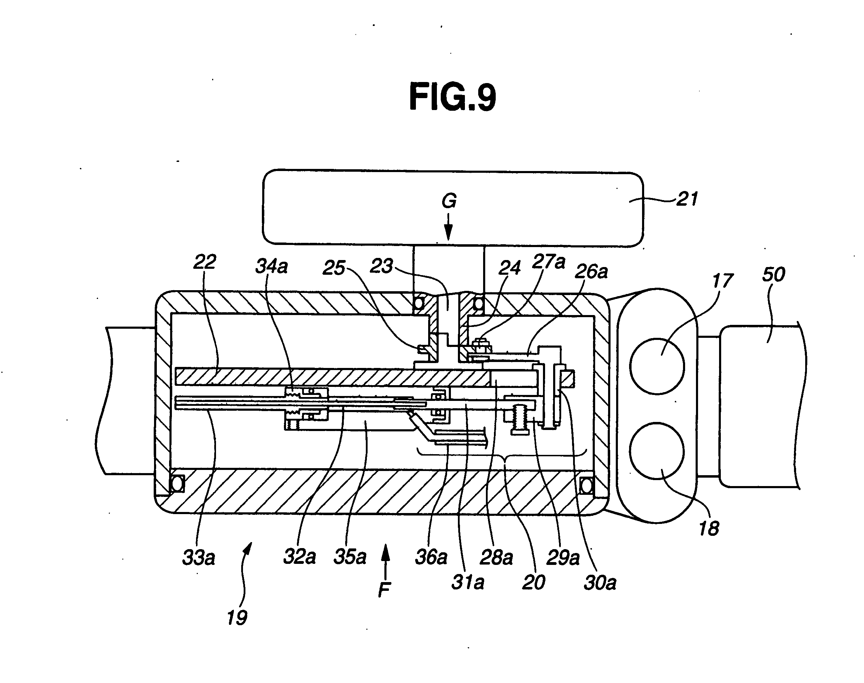

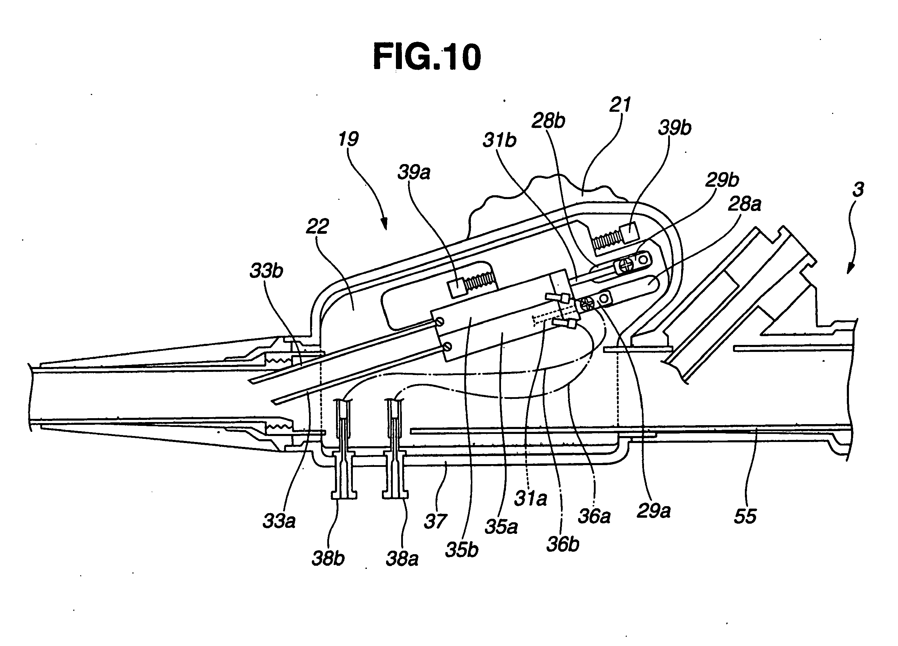

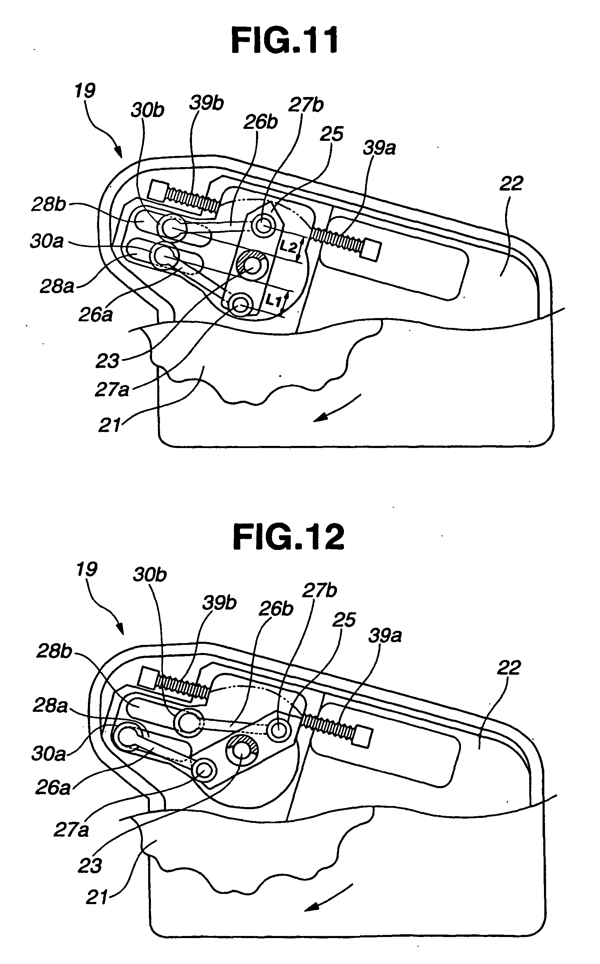

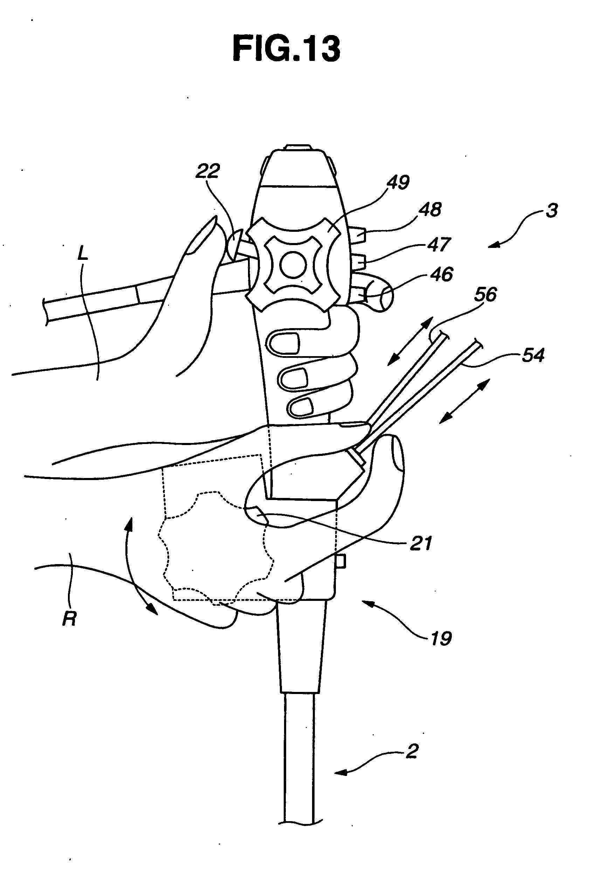

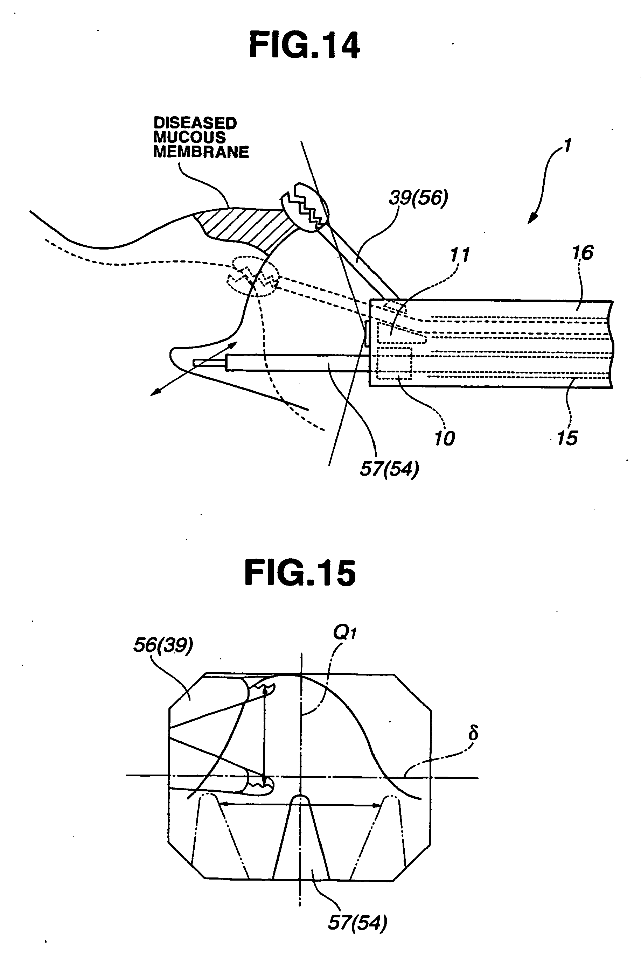

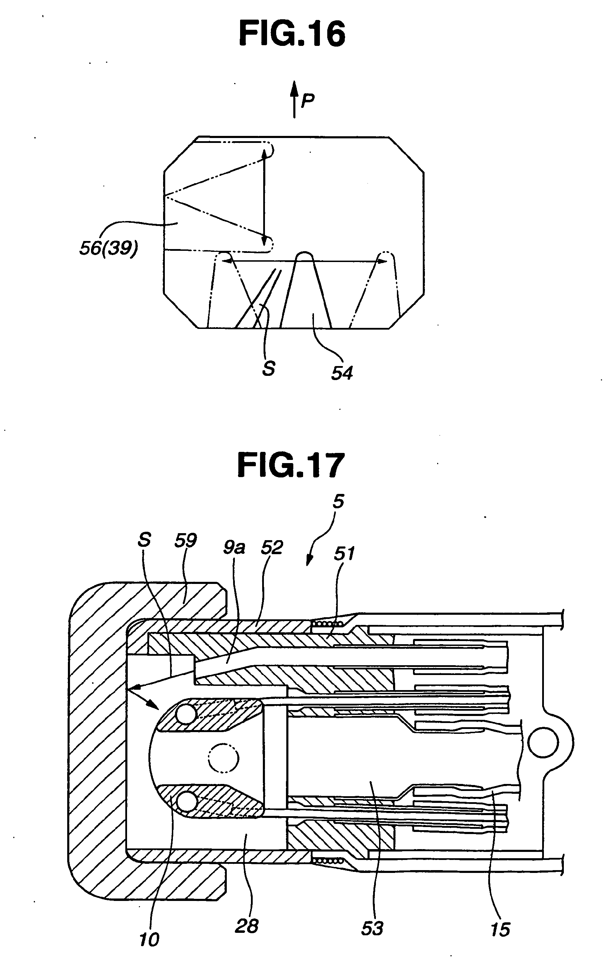

Fig. 1 is a perspective view showing an overall configuration of an endoscope according to the present invention. Fig. 2 is a plan view showing a configuration of a distal end of an endoscope inserting portion of the endoscope. Fig. 3 is a perspective view showing the distal end of the endoscope inserting portion. Fig. 4 is a plan view showing a distal end component except a front face of an electric insulating cover of the distal end of the endoscope inserting portion. Fig. 5 is a perspective view showing the configuration of a first instrument swing table provided at the distal end of the endoscope inserting portion. Fig. 6 is a sectional view showing a cross-section cut at a VI to VI line of Fig. 2 of the distal end of the endoscope inserting portion for the sake of describing operation. Fig. 7 is a sectional view showing a relation between a first instrument swing table and an instrument provided at the distal end of the endoscope inserting portion, which is cut at a VII to VII line of Fig. 2. Fig. 8 is a sectional view showing the cross-section of the endoscope inserting portion for the sake of describing the operation as with Fig. 7. Fig. 9 is a sectional view showing an internal configuration of a first swing table operating portion provided to an endoscope operating portion. Fig. 10 is a plan view viewed from an F direction of Fig. 9 and showing the internal configuration of the first swing table operating portion provided to the endoscope operating portion. Fig. 11 is a plan view viewed from a G direction of Fig. 9 and showing the internal configuration of the first swing table operating portion provided to the endoscope operating portion. Fig. 12 is a plan view showing a state of having rotationally driven from the state of Fig. 11 and shows the internal configuration of the first swing table operating portion provided to the endoscope operating portion. Fig. 13 is an explanatory diagram for describing an operational state of the endoscope. Fig. 14 is an explanatory diagram for describing a swinging state of the instrument in an endoscope image of the endoscope. Fig. 15 is an explanatory diagram for describing a swinging operation of the instrument in the endoscope image. Fig. 16 is an explanatory diagram for describing a cleaning state by the endoscope inserting portion in the endoscope image. Fig. 17 is a sectional view showing the cleaning state of the distal end of the endoscope inserting portion.

First, the overall configuration of the endoscope according to the present invention will be described by using Fig. 1. An endoscope 1 according to the present invention mainly comprises an inserting portion 2, an operating portion 3 provided at a base end of the inserting portion 2 and a universal cord 4 having a light guide and the like connected to the operating portion 3 inserted - therein.

The inserting portion 2 comprises a flexible tube portion 12 formed by a slender flexible member, a bending portion 14 bending vertically and horizontally and the distal end 5 in order from the base end. The distal end 5 includes a distal end component 51 which is provided with observation means 6, two illumination windows 7, 7, an air and water nozzle 8 for supplying air and water to the observation means 6, a front water supply port 9, a first instrument swing table 10 and a second instrument swing table 11.

The inserting portion 2 is provided with a first instrument insertion channel 15 (refer to Fig. 2) and a second instrument insertion channel 16 (refer to Fig. 2) therein though not shown in Fig. 1. A tip of the first instrument insertion channel 15 opens at the first instrument swing table 10 provided on the distal end 5, and a rear end thereof is communicated with a first opening 17 provided on the operating portion 3. The tip of the second instrument insertion channel 16 opens at the second instrument swing table 11 provided on the distal end 5, and the rear end thereof is communicated with a second opening 18 provided on the operating portion 3.

The operating portion 3 incorporates a first swing table operating knob 21 configured to remotely operate the first instrument swing table 10 provided on the base side of the inserting portion 2, a first swing table operating portion 19 containing a first instrument swing table operating mechanism 20 (refer to Fig. 9) described later configured to swing the first instrument swing table 10 on operating the first swing table operating knob 21, the first opening 17 and second opening 18 placed as close as possible to the first swing table operating portion 19 and provided on the upside of the first swing table operating portion 19 in the drawing, a gripper 50 provided on the upside of the first opening 17 and second opening 18 in the drawing for an operator to grip the operating portion 3 and a second swing table operating function not shown provided on the upside of the gripper 50 in the drawing configured to remotely operate the second instrument swing table 11, where a second swing table operating knob 45 as a part of the second swing table operating function is provided outside.

Outside the operating portion 3 where the second swing table operating knob 45 is provided, there are an air and water supply control button 46, a suctioning control button 47, an image recording button 48, a bending operating knob 49 and the like. The second swing table operating knob 45 is operated by a left thumb of the operator gripping the gripper 50, and the first swing table operating knob 21 is provided on the same surface side where the bending operating knob 49 is provided.

The first swing table operating knob 21 is provided as close as possible to the first opening 17 on the same surface side where the bending operating knob 49 is provided. The first swing table operating knob 21 is normally operated by a right hand. To be more specific, as for the endoscope 1, the air and water supply control button 46, the suctioning control button 47, the image recording button 48 and the bending operating knob 49 are operated while operating the second swing table operating knob 45 configured to remotely operate the second instrument swing table 11 by gripping the gripper 50 of the operating portion 3 by a left hand LH of the operator (refer to Fig. 16). A right hand RH of the operator performs a forward/backward movement operation of a first instrument 54 and a second instrument 56 inserted through the first instrument insertion channel 15 and the second instrument insertion channel 16 from the first opening 17 and the second opening 18, an operation of the first swing table operating knob 21 and forward/backward movement and twisting operations of the inserting portion 2.

The configuration of a distal end face of the distal end 5 of the endoscope 1 will be described by using Fig. 2. As previously described, the distal end face of the distal end 5 is provided with the observation means 6 approximately at the center of the front face, the two illumination windows 7 placed sandwiching the observation means 6, the air and water nozzle 8 configured to supply air and water to the observation means 6, a front water supply port 9 configured to supply water to the front from the distal end 5, the first instrument swing table 10 and the second instrument swing table 11.

The observation means 6 of the present embodiment comprises an observation optical system and a solid-state image sensing device placed at a focal position of the observation optical system. An arrow P direction in the drawing is an upward direction of a screen of an endoscope image displayed on a monitor not shown based on an image pickup signal of an observed region of which image was picked up by the solid-state image sensing device.

The first instrument swing table 10 is communicated with the first instrument insertion channel 15 and horizontally swung in the drawing by two operating wires 32a, 32b. The second instrument swing table 11 is communicated with the second instrument insertion channel 16 and vertically swung in the drawing by one operating wire 32c. The first instrument swing table 10 is housed in the opening provided only on the front face of the distal end face of the distal end 5 while the second instrument swing table 11 is housed in the opening provided on the front face to a side face of the distal end face of the distal end 5.

The opening on the side face of the distal end 5 is opened in a minimum size not to interfere with a swinging instrument or the second instrument swing table 11. The first instrument swing table 10 is provided with a first instrument inserting portion 40 configured to insert the instrument inserted from the first instrument insertion channel 15. The second instrument swing table 11 also has the instrument inserting portion configured to insert the instrument inserted from the second instrument insertion channel 16 formed thereon.

The distal end 5 is rigidly formed by a metallic member. An external surface of the distal end 5 is almost entirely covered by an electric insulating cover 52 formed by an electric insulating member. The electric insulating cover 52 is formed in a shape partially swollen outside so as not to contact the first instrument swing table 10 when swinging.

Furthermore, the observation means 6 and the first instrument swing table 10 are placed so that a center line Q equally dividing the field of view of the observation means 6 into the right and left and a rotation axis R of the first instrument swing table 10 approximately match horizontally when viewed from the front of the distal end 5.

A fluid delivery passage 9a configured to deliver a fluid in an observation direction in front of the distal end 5 is placed on the right side of the first instrument swing table 10 in the drawing and below the second instrument swing table 11 in the drawing. As shown in Fig. 3, a delivery direction of the fluid delivery passage 9a is approximately parallel with a horizontal swinging direction of the first instrument swing table 10 in the drawing when viewed from the front of the distal end 5, which is formed to be directed to the center line Q equally dividing the field of view of the observation means 6 into the right and the left.

To give a detailed description as to a vertical relation between the opening position of the fluid delivery passage 9a and the first instrument swing table 10 in a perpendicular direction in the drawing in particular as against the swinging direction by using Figs. 1 and 3, an opening width 11 of the fluid delivery passage 9a in an approximately perpendicular direction to the swinging direction of the first instrument swing table 10 is placed within a width of a vertical swinging range of the instrument when the instrument projected from the first instrument swing table 10 is horizontally swung, that is within a vertical width 12 of the first instrument inserting portion 40 of the first instrument swing table 10.

The delivery direction of the fluid delivery passage 9a is set up to pass inside an instrument swinging area indicated by oblique lines in the drawing on horizontally swinging the instrument projected from the first instrument inserting portion 40 of the first instrument swing table 10 so as to be a water supply direction line S1 which is directed to the center line Q equally dividing an observation field of view of the observation means 6 into the right and left. To be more specific, when viewed from the front of the distal end 5, the water supply direction line S1 from the fluid delivery passage 9a has its delivery direction set up to be within projected area (shaded area in the drawing) on which the instrument swinging area is projected in front from the distal end face of the distal end 5.

A dashed line G in the drawing in Fig. 2 indicates the shape of a first channel opening 53 described later when viewed from the front, which is an oval shape longer in a horizontal direction than in a vertical direction.

Next, a description will be given by using Fig. 4 as to housing the first instrument swing table 10 and the second instrument swing table 11 into the distal end component 51 of the distal end 5.

Fig. 4 is a plan view showing a state of removing the electric insulating cover 52 covering the front face of the distal end component 51 shown in Fig. 2.

The distal end component 51 is provided with a first storage room 28 configured to house the first instrument swing table 10 and a second storage room 29 configured to house the second instrument swing table 11. The first storage room 28 and the second storage room 29 are coupled in a C portion close to the fluid delivery passage 9a in the drawing. The first storage room 28 and the second storage room 29 are coupled in the C portion to facilitate cleaning of the first and second instrument swing tables 10, 11 by a cleaning method described later.

The configuration of the first instrument swing table 10 will be described by using Fig. 4. The first instrument swing table 10 is inserted into a member formed in an overall shape being approximately columnar or elliptic-cylindrical or in the shape of a rectangular column having its one side formed as an arc from the first opening 17 of the operating portion 3 so as to form the first instrument inserting portion 40 from which the instrument inserted into the first instrument insertion channel 15 is led out.

A rotation axis hole 42 in which a rotation axis not shown is to be mounted is formed at the center of an undersurface 41 of the first instrument swing table 10. The undersurface 41 and a top surface 43 of the first instrument swing table 10 have notched faces which are notched in mutually different directions formed thereon. One of the notched faces has a wire terminal member 44a of the operating wire 32a rotatably mounted thereon while the other notched face has a wire terminal member 44b of the operating wire 32b rotatably mounted thereon. To be more specific, the two operating wires 32a, 32b are pulled so that the first instrument swing table 10 can rotate from left to right or right to left in the drawing in reference to the rotation axis hole 42.

The second instrument swing table 11 has approximately the same configuration as that of the first instrument swing table 10, which can be rotated vertically in the drawing by the operating wire 32c.

Here, a description will be given by using Fig. 6 as to the relation between the second instrument swing table 11 and the instrument inserted into the second instrument insertion channel 16. Fig. 6 is a sectional view cut at a VI to VI line of Fig. 1 and viewed from the arrow direction in the drawing.

The second instrument swing table 11 is rotatably mounted in proximity to the tip of a second channel opening 55a to which the second instrument insertion channel 16 is connected. At the center of the second instrument swing table 11, a second instrument inserting portion 58 into which the second instrument 56 is to be inserted is formed. The second instrument 56 inserted into the second instrument inserting portion 58 and projected into a body cavity from the distal end face of the distal end 5 swings vertically to the range indicated by a two-dot chain line in the drawing by remotely controlling the operating wire 32c. An amount of projection U of the second instrument 56 from the distal end face of the distal end 5 of the second instrument 56 is a distance suitable for observability and processability of an affected region, such as 15 to 25 mm. The vertical swinging of the distal end of the second instrument 56 should not exceed a vertical observation field of view range Y of the observation means 6.

A setup is made so that the distal end of the second instrument 56 does not reach a height of the horizontal swinging of the first instrument 54 when the second instrument swing table 11 is completely inversed. To be more specific, it is set up in a positional relation in which the first instrument 54 does not contact the second instrument 56 even if the first instrument 54 is horizontally swung by the first instrument swing table 10 in the state of having the second instrument swing table 11 completely inversed and having the second instrument 56 projected straight in an axial direction of the distal end 5.

Next, a description will be given by using Figs. 7 and 8 as to the relation between the first instrument swing table 10 and the instrument inserted into the first instrument insertion channel 15. Figs. 7 and 8 are sectional views cut at a section line VII to VII shown in Fig. 2.

The first instrument swing table 10 is provided to the first channel opening 53 connected to the distal end of the first instrument insertion channel 15 which is communicated with the first opening 17 of the operating portion 3 and contained in the flexible tube portion 12, the bending portion 14 and to the distal end 5, and it is rotatably mounted by mounting the rotation axis not shown in the rotation axis hole 42.

The first instrument 54 inserted from the first opening 17 and inserted into the first instrument insertion channel 15 and the first channel opening 53 is led out to the affected region in the body cavity located in front of the distal end 5 via the instrument inserting portion 40 of the first instrument swing table 10. If the operating wires 32a, 32b of the first instrument swing table 10 having led out the first instrument 54 are pulled by the remote operation, the first instrument 54 horizontally swings in the range indicated by the two-dot chain line in the drawing.

As for the horizontal swinging range of the first instrument swing table 10, however, the distal end of the first instrument 54 being swung should not exceed a field of view range F of the observation optical system of the observation means 6. As will be described in detail later in the description of the configuration and action of the first swing table operating portion 19 of the operating portion 3, the swinging of the first instrument swing table 10 is set up so that, when the first swing table operating knob 21 is operated at an even rotation speed, the swinging speed of the distal end of the first instrument 54 is faster in the case where the distal end of the first instrument 54 is at the center of the field of view range of the observation optical system than in the case where it is in both the peripheral parts of the field of view range of the observation optical system.

Next, a description will be given by using Figs. 9 to 12 as to the first swing table operating portion 19 provided on the operating portion 3 configured to swing the first instrument swing table 10 by remote operation.

As shown in Fig. 9, the first swing table operating portion 19 is provided with the first instrument swing table operating mechanism 20 inside it and provided with the first swing table operating knob 21 which is also a part of the first instrument swing table operating mechanism 20 outside it.

The first instrument swing table operating mechanism 20 of the first swing table operating portion 19 includes a swing table operating portion body 22 as a structure and a rotation axis 23 fixed on the swing table operating portion body 22. On the outside of the rotation axis 23, an operating knob rotation cylinder 24 fixed on the first swing table operating knob 21 is fitted. The operating knob rotation cylinder 24 is rotatably fitted to the rotation axis 23. The operating knob rotation cylinder 24 has a rotating plate 25 fitted to the distal end thereof. The rotating plate 25 is also rotatable against the rotation axis 23. To be more specific, if the first swing table operating knob 21 is rotatively operated, the rotation of the first swing table operating knob 21 is transmitted to the rotating plate 25 via the operating knob rotation cylinder 24.

To the rotating plate 25, one ends of two L-shaped rods 26a, 26b are rotatably fitted by wheel pins 27a, 27b (refer to Figs. 11 and 12). The other ends of the rods 26a, 26b are inserted into guide holes 28a, 28b (refer to Figs. 11 and 12) formed on the swing table operating portion body 22 to be rotatably fitted to brackets 29a, 29b (refer to Fig. 10). The guide holes 28a, 28b provided on the swing table operating portion body 22 are linear slits formed in a longitudinal direction of the swing table operating portion body 22, where the rods 26a, 26b are mounted via friction reduction members 30a, 30b (refer to Figs. 11 and 12) to be able to move inside the guide holes 28a, 28b back and forth.

To be more specific, it is configured that, if the rotation of the first swing table operating knob 21 is transmitted to the rotating plate 25, the rods 26a, 26b are moved in the guide holes 28a, 28b of the swing table operating portion body 22 by rotational motion of the rotating plate 25 so as to move the brackets 29a, 29b in a straight line.

The brackets 29a, 29b have wire joining members 31a, 31b detachably fixed thereon by screws as shown in Fig. 10. The wire joining members 31a, 31b have the ends of the two operating wires 32a, 32b from the first instrument swing table 10 of the distal end 5 connected and fixed thereto by solder or silver solder. The operating wires 32a, 32b are covered by operating wire guide tubes 33a, 33b respectively. The tips of the operating wire guide tubes 33a, 33b are connected to the distal end 5 in which the first instrument swing table 10 is housed in the form of opening respectively (refer to Fig. 7).

The base sides of the wire guide tubes 33a, 33b are detachably fitted and fixed to the tips of cylinders 35a, 35b by keeping them watertight via guide tube holding members 34a, 34b provided on the first swing table operating portion 19. The cylinders 35a, 35b are provided on the swing table operating portion body 22 to move the wire joining members 31a, 31b in a straight line.

The swing table operating portion body 22 is provided to an operating portion structure 55 of the operating portion 3 as shown in Fig. 10. On the top surface of the swing table operating portion body 22, there are the two cylinders 35a, 35b fixed in parallel, into which the two operating wires 32a, 32b configured to remotely operate the first instrument swing table 10 are inserted respectively. A joining section of the wire joining members 31a, 31b and the brackets 29a, 29b is placed in a space different form the space connecting the inserting portion 2 with the operating portion 3. The cylinders 35a, 35b are fitted with cleaning water supply tubes 36a, 36b for cleaning the inside of the operating wire guide tubes 33a, 33b including the operating wires 32a, 32b. The other ends of the cleaning water supply tubes 36a, 36b are connected to cleaning bases 38a, 38b provided on an exterior member 37 of the swing table operating portion 19. The cleaning bases 38a, 38b can be connected with a syringe (not shown) so as to send cleaning fluid and the like into the operating wire guide tubes 33a, 33b.

Next, a description will be given by using Figs. 11 and 12 as to the relation between the rotating plate 25 of which rotation center is the rotation axis 23 and the two rods 26a, 26b.

The rotating plate 25 centers on the rotation axis 23, and has one ends of the rods 26a, 26b rotatably mounted at symmetrical positions by the wheel pins 27a, 27b. If the first swing table operating knob 21 is turned, the rotating plate 25 turns in conjunction and the rods 26a, 26b are moved in a straight line by the guide holes 28a, 28b, which pulls one of the operating wires 32a, 32b connected to the brackets 29a, 29b provided on the other ends of the rods 26a, 26b and pushes out the other by almost the same amount. The swing table operating portion body 22 is provided with stoppers 39a, 39b for adjusting a rotation range of the rotating plate 25. It is possible, by adjusting the stoppers 39a, 39b, to regulate the rotation range of the rotating plate 25 and thereby adjust the rotation range of the first instrument swing table 10.

As for the rotational position of the rotating plate 25 shown in Fig. 11, the first instrument swing table 10 is in a rotational state shown in Fig. 7, where the distal end of the first instrument 54 led out of the first instrument swing table 10 is positioned at the center of the field of view.

In the state of the rotational position of the rotating plate 25 in Fig. 11, the rods 26a, 26b are set at the position for maximizing distances L1, L2 (L1 = L2) shown in the drawing to the rotation center of the wheel pins 27a, 27b connecting the rotating plate 25 with the rods 26a, 26b in the direction approximately orthogonal to the axes in the respective directions for moving in a straight line in the rotation range of the rotating plate 25 (indicated by the two-dot chain line in the drawing).

To be more specific, if the first swing table operating knob 21 is turned at the same speed, the speed of the linear motion of the rods 26a, 26b moving in the guide holes 28a, 28b becomes fastest when the rotating plate 25 reaches the position shown in Fig. 11. If the rotating plate 25 is further turned from the position of the rotating plate 25 shown in Fig. 11, the speed of the linear motion of the rods 26a, 26b moving in the guide holes 28a, 28b gradually decreases so that the rotating plate 25 turns to the position shown in Fig. 12. To be more specific, the rotation speed of the first instrument swing table 10 is slowed down by operating from the state shown in Fig. 11 to the state shown in Fig. 12.

The rotational position of the rotating plate 25 shown in Fig. 12 is the state where the distal end of the first instrument 54 projected from the first instrument swing table 10 shown in Fig. 7 is A, that is, the state where it is located in the peripheral part (fully on the right side on the endoscope image) of the field of view shown in Fig. 8.

It is also possible to turn the.rotating plate 25 at a constant speed with an electric motor instead of manually turning the first swing table operating knob 21.

A description will be given by using Figs. 13 to 16 as to a mucous membrane incision process of the affected region with the instrument based on endoscope observation in the body cavity by using the endoscope of such a configuration.

First, as shown in Figs. 13 and 14, a high-frequency knife 57 as the first instrument 54 is inserted into the first instrument insertion channel 15 (not shown in Fig. 13), and a grip forceps 39 as the second instrument 56 is inserted into the second instrument insertion channel 16 (not shown in Fig. 13). And the first and second instrument swing tables 10, 11 are swung by the first swing table operating knob 21 and the second swing table operating knob 45 so as to incise the affected region.

To begin with, the instrument including an injection needle is inserted into the first instrument insertion channel 15 of the inserting portion 2 of the endoscope 1 inserted to the proximity of the affected region in the body cavity to inject a drug solution such as normal saline into diseased submucosa so as to upheave the entire mucosal layer including the diseased mucous membrane.

Next, as indicated by the dotted line in Fig. 14, the diseased mucous membrane or its periphery is gripped by using the grip forceps 39 which is the second instrument 56 projected from the second instrument insertion channel 16. The grip forceps 39 is raised by the second instrument swing table 11 as shown in solid line in the drawing to lift the mucous membrane including the affected area.

Next, the high-frequency knife 57 as the first instrument 54 is inserted into the first instrument insertion channel 15 as a replacement, and the first instrument swing table 10 is horizontally swung. And the high-frequency knife 57 is put in contact with a foot or the submucosa of the diseased mucous membrane lifted by the grip forceps 39 to energize and incise it while horizontally swinging it.

A description will be given by using Fig. 15 as to the swinging operation of the high-frequency knife 57 and the grip forceps 39 on the endoscope image of the observation means 6.

The endoscope image of the affected region by the observation optical system of the observation means 6 shown in Fig. 15 shows the state where the mucous membrane of the affected region is raised upward by the grip forceps 39 vertically swinging on the screen with the second instrument swing table 11 and the state where the high-frequency knife 57 as the first instrument 54 is located on the downside of the mucous membrane of the affected region raised by the grip forceps 39 to incise the submucosa of the affected region by horizontally swinging it with the first instrument swing table 10.

When incising the affected region raised by swinging the grip forceps 39 with the second instrument swing table 11 based on the endoscope image, the first swing table operating knob 21 is turned at a constant speed, and the rotating operation (rotational motion) of the first swing table operating knob 21 is converted to linear motion for pulling the operating wires 32a, 32b by the first swing table operating portion 19. And the high-frequency knife 57 is horizontally swung by using the first instrument swing table 10 through the linear motion of the operating wires 32a, 32b. In this case, it is possible to render the swinging speed of the high-frequency knife 57 faster in proximity to a screen center Q of the endoscope image and render it slower in both the peripheral parts of the endoscope image than at the screen center Q.

The endoscope image shown in Fig. 15 is an image obtained from a placement relation between the first instrument swing table 10 and the second instrument swing table 11 as against the observation means 6 shown in Fig. 2. As for the placement relation among the observation means 6, the first instrument swing table 10 and the second instrument swing table 11, the first instrument swing table 10 and the second swing table 10 operating knob may be placed by turning them clockwise by 90 degrees against the center of the observation means 6 for instance. In this case, the endoscope image of Fig. 15, when viewed after counterclockwise rotation by 90 degrees, has the high-frequency knife 57 as the first instrument 54 shown on the right side of the screen of the endoscope image to swing vertically on the screen. The swinging speed of the high-frequency knife 57 in this case is fastest in proximity to a center line δ equally dividing the screen of the endoscope image vertically. Meanwhile, the grip forceps 39 is shown on the downside of the screen of the endoscope image and horizontally swings on the screen.

As described above, the affected region is generally picked up at the center of the screen of the endoscope image to perform cure and treatment. The peripheral part of the screen of the endoscope image is hard to see because it is not a front view, and the image looks distorted due to influence of an aberration of the observation optical system and the like. Thus, in the case of incising the mucous membrane of the affected region shown in the peripheral part of the screen of the endoscope image with the incision instrument such as the high-frequency knife, it is necessary to pay attention to the swinging speed of the instrument in particular.

As for the endoscope of the first embodiment of the present invention in comparison, it has a control mechanism of the first instrument swing table operating mechanism 20 so that, when it is rotatively operated at a constant speed by the swing table operating portion including the first swing table operating knob 21 configured to remotely operate on the user's side the first instrument swing table 10 configured to operate with swing table the first instrument 54 such as the high-frequency knife 57 which is an incision instrument, the swinging speed of the first instrument 54 swung by the first instrument swing table 10 is faster at the center of the screen of the endoscope image and slower in both the peripheral parts of the screen than at the center of the screen. Thus, it is possible to efficiently perform incision work of the affected region displayed at the center of the screen of the endoscope image and carefully perform safe incision work of the affected region displayed in the peripheral parts of the screen.

In the case of swinging with a distance to the affected region suitable for observability and curative processability of the first instrument 54 led out of the distal end face of the distal end 5 of the inserting portion 2, such as 15 to 25 mm, the fastest moving point of the instrument 54 is set up when the distal end of the first instrument 54 reaches the center line equally dividing the field of view of the endoscope image into the right and left (six o'clock direction of a clock according to the first embodiment). For this reason, the operator can easily recognize the fastest point and consequently perform the incision work more safely and efficiently.

The stoppers 39a, 39b configured to regulate the swinging range of the first instrument swing table 10 of the first swing table operating portion 19 is structured to regulate the rotation of the rotating plate 25 which is spatially formable with high strength rather than being provided on the thin rods 26a, 26b. Therefore, the stoppers 39a, 39b are deformed little by the impact of being frequently hit by the rotating plate 25. Consequently, the swinging range of the instrument is hardly reduced over time.

As for cleaning of the affected area on energization and incision of the submucosa of the diseased mucous membrane with the high-frequency knife 57 as the first instrument 54, it is performed with cleaning water delivered to the front from the fluid delivery passage 9a. The cleaning water after the cleaning is aspirated outward with an unshown suction pump by using the first instrument insertion channel 15.

As mentioned above, in general, a tissue of the mucous membrane sticks to a blade edge of the high-frequency knife 57 when incising the diseased mucous membrane with the high-frequency knife 57. If the incision process is continued in the state of having the tissue stuck thereon, the sticking mucous membrane carbonizes to blunt the high-frequency knife 57.

Thus, as shown in the endoscope image observed from the observation means 6, the distal ends of the first instrument 54 and the second instrument 56 swing in the range of the arrows in the drawing as they are swung by the first instrument swing table 10 and the second instrument swing table 11 described above, and the delivery from the fluid delivery passage 9a sends the solution to the high-frequency knife 57 as the water supply direction line S1. As for the operation, a description will be given by using Fig. 16 as to the operation of incising the diseased mucous membrane in the body cavity based on the observation by the endoscope image which shows the swinging of the first instrument 54 and the second instrument 56 and the water supply direction line S1. If the tissue of the incised mucous membrane sticks to the high-frequency knife 57, the high-frequency knife 57 is moved to the direction of the water supply direction line S1 of the fluid delivery passage 9a by the first instrument swing table 10 so as to deliver the fluid such as the cleaning water or the air from the fluid delivery passage 9a. It is possible, by means of the fluid from the fluid delivery passage 9a, to remove the tissue of the mucous membrane sticking to the high-frequency knife 57 which has been moved to the position of the water supply direction line S1 of the fluid delivery passage 9a.

To be more specific, if the delivery direction of the front water supply port 9 is the center line (center of the screen) Q equally dividing the field of view of the observation means 6 into the right and left, it is possible, by setting the water supply direction line S1 penetrating inside the swinging area of the first instrument swing table 10, to easily move the high-frequency knife 57 as the first instrument 54 on the endoscope image toward the water supply direction line S1 so as to allow the high-frequency knife 57 to be cleaned by the fluid delivered in front.

It is thereby possible to perform an incision action while alternately repeating the mucous membrane incision and cleaning in the state of having the high-frequency knife 57 inserted into the first instrument insertion channel 15. Therefore, the efficiency of incision operation improves.

Conventionally, in the case of cleaning a wide range of the affected area with the cleaning water delivered to the front from the fluid delivery passage 9a, the water was supplied in front while bending the bending portion 14 of the inserting portion 2. As for the endoscope of the present invention, however, if the first instrument 54 is swung by the first instrument swing table 10 while sending the cleaning water to the front from the fluid delivery passage 9a, the cleaning water sent from the fluid delivery passage 9a is sent by way of the surface of the first instrument 54. Therefore, the cleaning water sent through the narrow water supply direction line S1 from the fluid delivery passage 9a is diffused by the first instrument 54 to allow the cleaning of the wide range. Consequently, the cleaning of the wide range is possible by a simple operation of just manipulating the instrument swing table without performing a conventional bending operation.

The first instrument swing table 10 of the endoscope of the present invention has the first channel opening 53 connected to the distal end of the first instrument insertion channel 15 as described by using Fig. 8. The first channel opening is in an elliptic shape which is longer in the horizontal direction than in the vertical direction when viewed from the front as indicated by the dashed line G in the drawing of Fig. 2. For this reason, the first instrument 54 can swing at a wide angle without being sandwiched between the first instrument swing table 10 and the distal end component 51 so as to improve the efficiency of the incision work by the first instrument 54. An opening width W1 in the swinging direction of the first channel opening 53 is larger than an inside diameter W2 of the first instrument insertion channel 15. It is thereby possible to take a large deflection in the first channel opening 53 of the first instrument 54 swung by the first instrument swing table 10 so that the swinging range of the first instrument 54 can be expanded. As for the vertical opening width of the first channel opening 53, there is no influence on the swinging of the first instrument 54 even if it is rendered narrower than the swinging direction. Therefore, it is possible to render an outside diameter of the distal end 5 thinner.

Furthermore, the opening of the first storage room 28 in which the first instrument swing table 10 is housed is provided with a notched portion 30 with a part of the electric insulating cover 52 in an irregular shape. Therefore, it is possible to swing the first instrument 54 at a wide angle without putting the first instrument 54 and the first instrument swing table 10 in contact with the electric insulating cover 52. To be more specific, it is possible to provide the endoscope capable of a wide range of incision without increasing the outside diameter of the distal end 5 as much as possible.

Furthermore, the opening position of the fluid delivery passage 9a is provided at a location of a distance E from the distal end face of the distal end 5. Therefore, it is possible to secure the minimum distance E between an open end of the fluid delivery passage 9a and the mucous membrane by performing the delivery to the front in the state where the distal end 5 is in close contact with or adjacent to the mucous membrane so that a water supply pressure on the mucous membrane can be reduced.

And now, it is necessary to clean the endoscope once the observation in the body cavity and curative treatment using the instruments are finished. In the cleaning of the endoscope, it takes a lot of trouble to clean every part of the complicated shape and structure by using a cleaning brush when cleaning the distal end component 51 of the inserting portion 2 of the endoscope 1 having the complicated shape and structure and provided with the instrument swing table. If multiple instrument swing tables are provided, the shape and structure become further complicated and there are some portions where the cleaning brush cannot reach. Thus, the cleaning required a lot of labor and meticulous care.

Consequently, the endoscope provided here is the one capable of easily and securely performing the cleaning of the endoscope having the instrument swing table provided at the distal end of the inserting portion. As described above by using Fig. 4, the distal end component 51 of the distal end 5 of the inserting portion 2 of the endoscope 1 has the first storage room 28 and the second storage room 29 configured to house the first instrument swing table 10 and the second instrument swing table 11 formed in conjunction in a C portion in the drawing.

As shown in Fig. 17, a cleaning cap 59 is mountable on the periphery and the distal end face of the electric insulating cover 52 covering the periphery of the distal end component 51 of such shape and structure. The cleaning cap 59 is formed by an elastic material such as rubber, and is detachably mountable on the distal end face and the periphery of the distal end face side of the distal end 5.

When cleaning the distal end 5, if the cleaning water is sent from the fluid delivery passage 9a after mounting the cleaning cap 59, the cleaning water bounces off the cleaning cap 59 as indicated by the arrow in the drawing, circulates to the first storage room 28 of the first instrument swing table 10 and also circulates from the joining section of C through the first storage room 28 to the second storage room 29 having the second instrument swing table 11 housed therein.

To be more specific, if the cleaning water is sent from the fluid delivery passage 9a after mounting the cleaning cap 59 on the distal end face side of the distal end 5, the cleaning water is circulated by the cleaning cap 59 to the first and second storage rooms 28, 29 having the first and second instrument swing tables 10, 11 housed therein so as to allow the first and second instrument swing tables 10, 11 to be securely cleaned.

The cleaning water having cleaned the first and second instrument swing tables 10, 11 is drained by using the first and second instrument insertion channels 15, 16.

In Fig. 17, the cleaning cap 59 covers the entire end face of the distal end 5. However, the cleaning cap 59 has to have at least a position for bouncing the cleaning water sent to the front from the fluid delivery passage 9a, that is, a wall configured to bounce the cleaning water on the water supply direction line S1. The portions other than that may be in a shape having a partial opening. If in such a shape, the cleaning water having cleaned the insides of the first and second storage rooms 28, 29 can flow outside from the partial opening. Therefore, it is possible to make it harder for the cleaning water after the cleaning to go into another duct of the distal end 5.

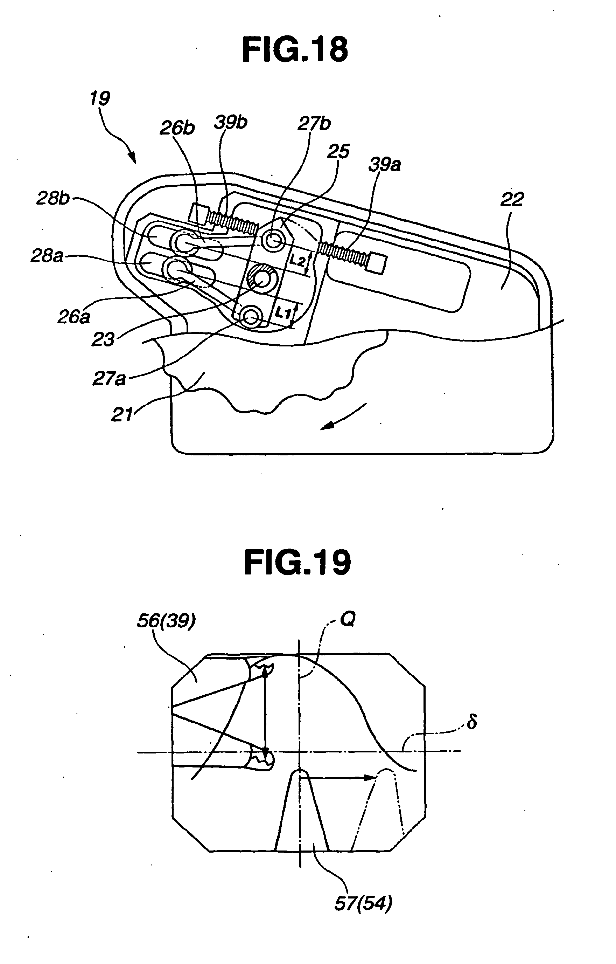

Next, a description will be given by using Figs. 18 and 19 as to the endoscope of a second embodiment of the present invention. Fig. 18 is a sectional view showing the configuration of the first instrument swing table operating portion of the endoscope, and Fig. 19 is an explanatory diagram for describing the action on the endoscope image screen of the instrument operated by the first instrument swing table operating portion of the endoscope. The same portions as those in Figs. 1 to 17 are given the same symbols, and a detailed description thereof will be omitted.

The endoscope of the second embodiment is basically the same as the above-mentioned first embodiment, where it is possible to set the swinging range of the first instrument swing table 10 in the first swing table operating portion 19 to be different.

To be more precise, as shown in Fig. 18, the rotation range of the rotating plate 25 of the first swing table operating portion 19 is regulated by the stoppers 39a, 39b to differentiate the horizontal swing range of the first instrument swing table 10.

As shown in the drawing, an adjustment is made by the stopper 39b to stop the guide hole 28b at the position for maximizing the distance L2 from a central axis on which the rod 26b moves in a straight line to the center of the wheel pin 27b rotatively fixing the rod 26b on the rotating plate 25.

Thus, an adjustment is made by the stopper 39b to stop the leftward rotation of the rotating plate 25 in the drawing at the position for maximizing the distance L2 between the center of the liner motion of the rod 26b and the center of the wheel pin 27b. And if the rotating plate 25 is rotated rightward in the drawing, the distance L2 becomes rotatable in the rotation range as indicated by the two-dot chain line in the drawing as in the above-mentioned first embodiment.

Thus, if an adjustment is made by the stoppers 39a, 39b as to the rotation range of the rotating plate 25, the horizontal swinging on the screen of the high-frequency knife 57 as the first instrument 54 is one-sided swinging only rightward in the drawing from the center line (center of the screen) Q equally dividing the field of view of the endoscope image into the right and left on the screen of the endoscope image observed by the observation optical system of the observation means 6 as shown in Fig. 11. In the one-sided swinging, it is possible to render the swinging speed faster in proximity to the center line Q of the screen of the endoscope image and render it slower in the peripheral parts of the screen of the endoscope image than at the center of the screen.

Consequently, the swinging of the first instrument 54 is only one-sided. However, it has the same action and effects as those of the aforementioned first embodiment.

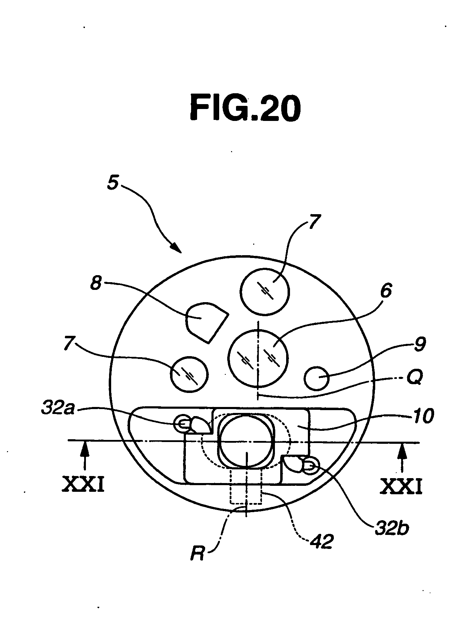

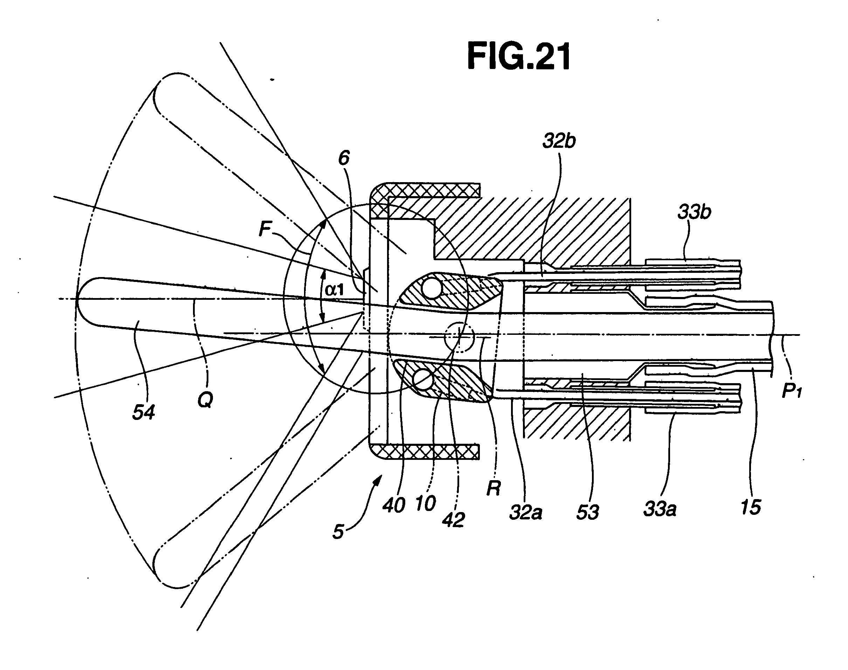

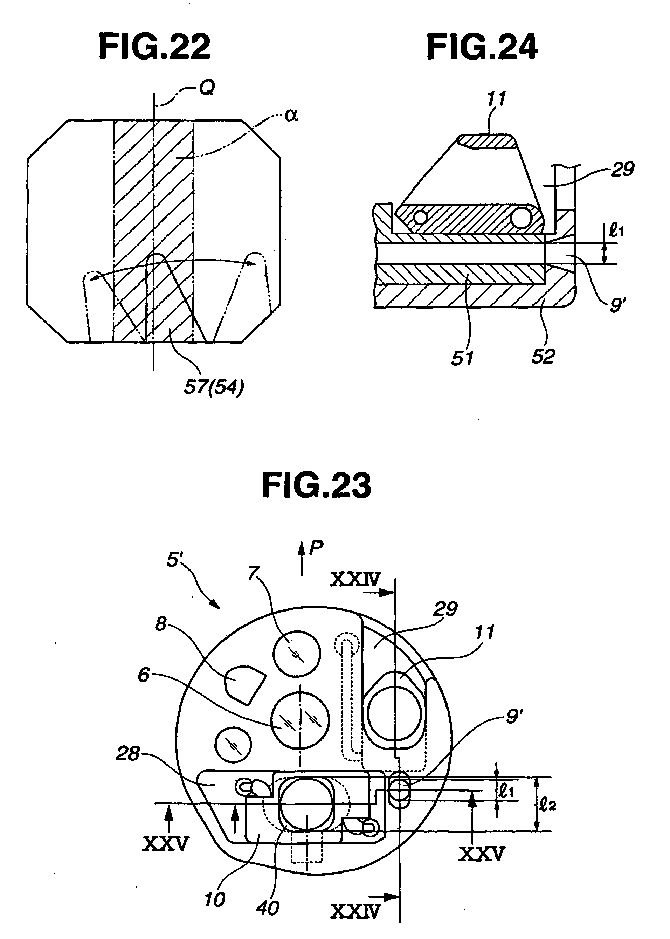

Next, the endoscope of a third embodiment according to the present invention will be described by using Figs. 20 to 22. Fig. 20 is a plan view showing the configuration of the distal end face of the distal end of the endoscope inserting portion, Fig. 21 is a sectional view cut at a section line XXI to XXI of Fig. 20 and showing the configuration of the distal end of the endoscope inserting portion, and Fig. 22 is an explanatory diagram for describing the screen of the endoscope image observed by the endoscope. The same portions as those in Figs. 1 to 19 are given the same symbols, and a detailed description thereof will be omitted.

As shown in Fig. 20, only the first instrument swing table 10 is provided as the instrument swing table on the distal end face of the distal end 5 of the endoscope inserting portion according to the third embodiment. As only the first instrument swing table 10 is provided at the distal end 5, the rotation axis R of the first instrument swing table 10 does not horizontally match with the center line Q equally dividing the field of view of the observation means 6 into the right and left when viewed from the distal end front of the distal end 5. As shown in Fig. 21, a central axis P1 of the first instrument 54 inserted into the first instrument insertion channel 15 and straightly led out to the front of the distal end 5 by way of the instrument inserting portion 40 of the first instrument swing table 10 does not horizontally match with the rotation axis R of the first instrument swing table 10 when viewed from the distal end front of the distal end 5.

Thus, in the case where the center line Q in the field of view direction of the observation means 6 and the central axis P1 of the first instrument 54 straightly led out do not horizontally match with the rotation axis R of the first instrument swing table 10 when viewed from the distal end front of the distal end 5, the position of the first instrument swing table 10 according to the first swing table operating portion 19 is set up so that the distal end of the first instrument 54 led out of the first instrument swing table 10 is on the center line Q of the field of view of the observation means 6 as indicated in solid line in Fig. 21. To be more specific, a setup is made so that, when the distal end of the first instrument 54 shown in Fig. 21 is positioned on the center line Q of the field of view of the observation means 6, the distance L1 (= L2) becomes maximum in the rotation range indicated by the rotating plate 25 of the first swing table operating portion 19 in Fig. 11.

An angle α1 in the drawing of Fig. 21 is a central area in the horizontal direction against the entire horizontal field of view S of the observation means 6, and is equivalent to approximately 1/4 (α = F/4) of the entire field of view F.

According to such a configuration, if the first swing table operating knob 21 is turned at a constant speed, it is possible, with the first swing table operating portion 19, to let the distal end of the first instrument 54 increase the swinging speed in a central area α1 of the field of view of the observation means 6 and reduce the swinging speed in the peripheral parts beyond the central area α1 according to the swinging of the first instrument swing table 10.

To be more specific, as to the screen of the endoscope image observed from the observation means 6, the high-frequency knife 57 as the first instrument 54 can increase the swinging speed in the shaded central area α1 in the horizontal direction of the field of view on the screen of the endoscope image and reduce the swinging speed in the peripheral parts of the screen on the right and left outside the central area α1 to be slower than the central area α1. Consequently, it is possible to have the same action and effects as those of the aforementioned first embodiment.

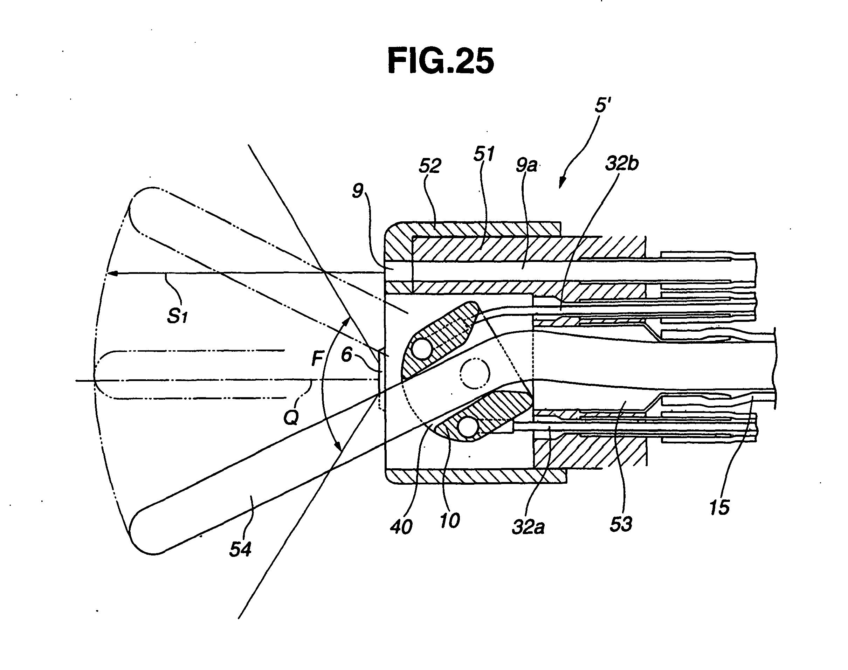

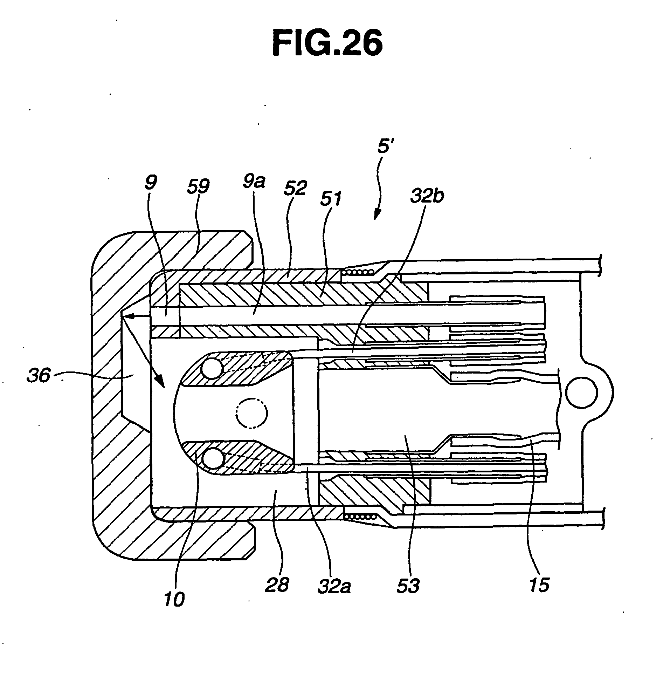

Next, a description will be given by using Figs. 23 to 26 as to the endoscope of a fourth embodiment of the present invention. Fig. 23 is a plan view showing the configuration of the distal end of the endoscope, Fig. 24 is a sectional view showing the configuration of the distal end of the endoscope, which is cut at a section line XXIV to XXIV shown in Fig. 23. Fig. 25 is a sectional view showing the configuration of the distal end of the endoscope, which is cut at a section line XXV to XXV shown in Fig. 23, and Fig. 26 is a sectional view for describing a detergent action of the distal end of the endoscope. The same portions as those in Figs. 1 to 22 are given the same symbols, and a detailed description thereof will be omitted.

A distal end 5' of the endoscope according to the fourth embodiment has an opening shape and a front water supply direction of the fluid delivery passage 9a different from those provided to the distal end 5 of the above-mentioned first embodiment. As shown in Fig. 23, as for the opening position of the fluid delivery passage 9a provided to the distal end 5' of the second embodiment, the opening width 11 of the fluid delivery passage 9a in an approximately perpendicular direction to the swinging direction of the first instrument swing table 10 is placed within a width of a vertical swinging area of the instrument when the instrument projected from the first instrument swing table 10 is horizontally swung, that is, within a vertical width 12 of the first instrument inserting portion 40 of the first instrument swing table 10 as in the above-mentioned first embodiment.

The opening shape of the distal end face of the distal end 5' of the fluid delivery passage 9a' is formed in the elliptic shape in the vertical direction in the drawing when the distal end face of the distal end 5' is viewed from the front. To be more specific, the cross-sectional shape of the fluid delivery passage 9a' is formed in the shape vertically expanding toward the distal end face side of the distal end 5' in the drawing as shown in Fig. 24. To be more specific, the water supply from the fluid delivery passage 9a' has a water supply form in the elliptic shape of which opening shape is vertically long.

As shown in Fig. 25, the water supply direction line S1 of the fluid delivery passage 9a' is not set up to send water to the center line Q equally dividing the field of view of the observation means 6 into the right and left. Instead, it is set up to be approximately parallel therewith. To be more specific, as described by using Fig. 3, it has only to be set up to be the water supply direction line S1 for passing through the swinging area of the first instrument swing table 10. As the ellipse-shaped water is sent in the swinging area, it becomes possible to remove the mucous tissue sticking to the first instrument 54 swung by the first instrument swing table 10 and clean the affected area in the incision process.

It is also possible to mount the cleaning cap 59 shown in Fig. 26 on the distal end 5'. The cleaning cap 59 is mounted on the distal end face side of the distal end 5' on which the fluid delivery passage 9a' is open. Inside the cleaning cap 59, a concave portion 36 is formed in the portion opposed to the water supply direction line S1 of the fluid delivery passage 9a' of the distal end 5'. The concave portion 36 is partially overlapping the first storage room 28 of the first instrument swing table 10.

If the cleaning water is sent from the fluid delivery passage 9a' to the front with the cleaning cap 59 mounted, the cleaning water bounces off the concave portion 36 of the cleaning cap 59, circulates in the first storage room 28 to clean the first instrument swing table 10 and also circulates to the second storage room 29 to clean the second instrument swing table 11.

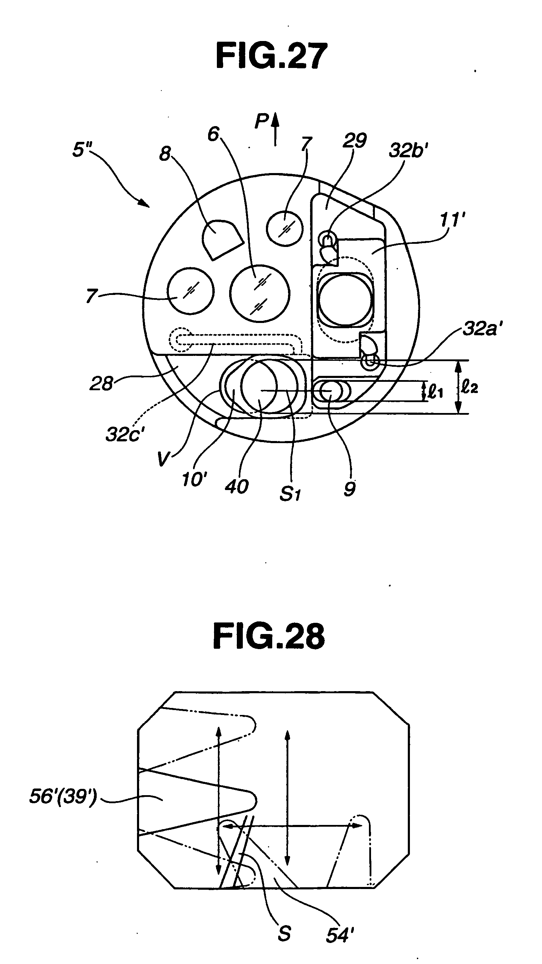

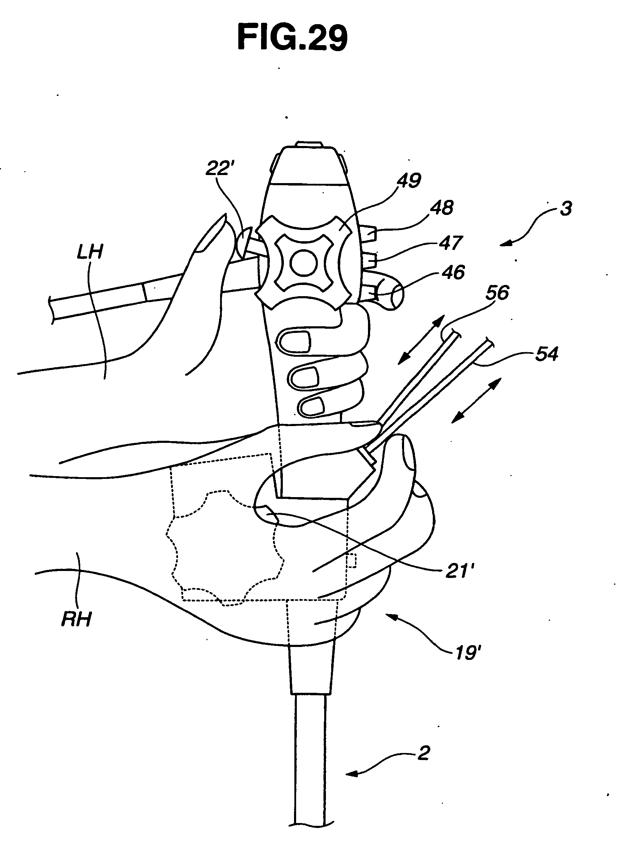

Next, a description will be given by using Figs. 27 to 29 as to the endoscope of a fifth embodiment of the present invention. Fig. 27 is a plan view showing the configuration of the distal end of the endoscope, Fig. 28 is an explanatory diagram for describing the swinging operation of the instrument in the endoscope image of the endoscope, and Fig. 29 is an explanatory diagram for describing the operation of the endoscope. The same portions as those in Figs. 1 to 26 are given the same symbols, and a detailed description thereof will be omitted.

As for the endoscope of the fifth embodiment, a first instrument swing table 10' and a second instrument swing table 11' are different from the above-mentioned first instrument swing table 10 and second instrument swing table 11. The endoscope of the fifth embodiment has the configuration in which the first and second instrument swing tables of the above-mentioned first embodiment are replaced.

As shown in Fig. 27, a distal end 5" of the endoscope of the fifth embodiment is provided with the first instrument swing table 10' horizontally swung by one operating wire 32c' and a second instrument swing table 11' vertically swung by two operating wires 32a', 32b'. The first instrument swing table 10' is swung by inserting a first instrument 54' which is a grip forceps while the second instrument swing table 11' is swung by inserting a second instrument 56' which is an incision instrument such as a high-frequency knife. It is the same as the first embodiment except for the first and second instrument swing tables 10' and 11'. In particular, the opening position of the fluid delivery passage 9a and the water supply direction line S1 are also the same as those described by using Fig. 3.

The first instrument inserting portion 40' of the first instrument swing table 10' is in a cylindrical shape covering the entire circumference of the first instrument 54' which is a grip forceps to be inserted not shown. However, it may also be in a partially notched shape. Reference character V in the drawing denotes the first channel opening 53. If the first instrument 54 is inserted into the first instrument inserting portion 40 in the state where the first instrument swing table 10 is inverted, the first instrument 54 projects by inclining to the fluid delivery passage 9a side when viewed from the front.

As for swinging motion of the first instrument 54' which is a grip forceps and the second instrument 56' which is a high-frequency knife projected from the first instrument swing table 10' and the second instrument swing table 11' respectively in the endoscope image picked up by the observation means 6 of the endoscope with the configuration of the distal end 5", they swing in the range of the arrows in the drawing and the water supply direction line S1 for sending the water from the fluid delivery passage 9a to the front is recognized as shown in Fig. 28.

The water supply direction line S1 sends the water to both the first instrument 54' and the second instrument 56'. To be more specific, the water supply direction line S1 is configured in the direction which penetrates the area in which the swinging area of the first instrument 54' intersects with the swinging area of the second instrument 56'.

The amount of projection from the distal end face of the distal end 5" of the first instrument 54' and the second instrument 56' is a distance suitable for observability and processability of the affected area, such as 15 to 25 mm.

Thus, it is possible to obtain the same action and effects as those of the aforementioned first embodiment even when the first instrument swing table and the second instrument swing table have different configurations.

The one operating wire 32c' of the first instrument swing table 10' of the distal end 5" is operated by a swing table operating portion 19' including a first swing table operating knob 21' of an operating portion 3' shown in Fig. 18 while the two operating wires 32a', 32b' of the second instrument swing table 11' is operated by the second swing table operating knob 45 of the operating portion 3'.

As for the operation of the endoscope in general, the first swing table operating knob 21 is provided at a position operable by a right hand on gripping a gripper of the operating portion 3 by a left hand while a second swing table operating knob 45 is provided at a position operable by the left hand as described by using Fig. 13.

When performing the incision process of the mucous membrane by operating the first swing table operating knob, 21 and the second swing table operating knob 45 and swinging the first instrument 54 and the second instrument 56 via the first instrument swing table 10 and the second instrument swing table 11, the high-frequency knife as an incision instrument is used as the first instrument 54 of the first instrument swing table 10 operated by the first swing table operating knob 21 while the grip forceps as a grip instrument is used as the second instrument 56 of the second instrument swing table 11 operated by the second swing table operating knob 21. For this reason, as for the incision process of the mucous membrane, there is an overwhelmingly high frequency of operating the first swing table operating knob 21 for swinging the high-frequency knife.

Meanwhile, in addition to the operation of the first swing table operating knob 21, the right hand needs to simultaneously perform the operation of inserting, advancing, retreating and twisting the inserting portion 2 in the body cavity and the operation of inserting, advancing and retreating the first instrument 54 and the second instrument 56 in the first instrument insertion channel 15 and the second instrument insertion channel 16 from the first opening 17 and the second opening 18.

In comparison, the left hand performs the operation of the second swing table operating knob 45, the operation of the air and water supply control button 46, the operation of the suctioning control button 47, the operation of the image recording button 48 and the operation of the bending operating knob 49 and the like of which frequencies of operation are relatively low. Thus, careful operations as well as highly frequent operations are required of the right hand.

Thus, the one operating wire 32c' configured to operate the first instrument swing table 10' of the distal end 5" described by using Fig. 27 is connected to the swing table operating portion 19' having the first swing table operating knob 21' provided thereon as shown in Fig. 29. And the two operating wires 32a', 32b' configured to operate the second instrument swing table 11' are connected to the swing table operating portion having the second swing table operating knob 21' provided thereon.

Thus, the right hand of high frequencies of various operations performs the operation of lifting the mucous membrane with the grip forceps of the first instrument 54' by the first instrument swing table 10' while the left hand performs a swing table operation of the high-frequency knife of the second instrument 56' by the second instrument swing table 11. Thus, the right hand can be dedicated to the operation of inserting, advancing, retreating and twisting the inserting portion 2 of the endoscope 1 in the body cavity and the operation of inserting, advancing and retreating the instrument, and the left hand performs the swing table operation of the high-frequency knife so that the incision work can be smoothly performed by the endoscope.

The present invention is not limited to the embodiments, but various other deformations may be implemented without departing from the scope thereof in an implementation phase. Furthermore, the embodiments include the inventions of various stages so that various inventions can be extracted by adequately combining multiple configuration requirements which are disclosed.

For instance, even if some of the configuration requirements are deleted out of all the configuration requirements indicated in the embodiments, the configuration having the configuration requirements deleted therefrom can be extracted as an invention in the case where the problems described in the means for solving the problem can be solved and the effects described in advantages of the invention can be obtained.

## Claims

1. An endoscope (1) comprising:
observation means (6) provided at a distal end (5) of an endoscope inserting portion (2);
an instrument insertion channel(16) provided to the endoscope inserting portion (2);
an instrument swing table (10) configured to swing an instrument inserted into the instrument insertion channel (16) and led out of the distal end of the endoscope inserting portion (2); and
a swing table operating portion (19) configured to remotely operate the instrument swing table (10) on a user's side;
**characterized by** a control mechanism (20) configured to control swinging of the instrument swing table (10) so that, when operating the swing table operating portion (19) at a constant speed, a swinging speed in the case where the distal end of the instrument led out of the distal end (5) of the endoscope inserting portion (2) is at a center (Q) of a screen of an endoscope image obtained by the observation means (6) is faster than the swinging speed in the case where the distal end of the instrument is in a peripheral part of the screen of the endoscope image.

2. The endoscope (1) according to claim 1, wherein the instrument swing table (10) is configured to swing the instrument inserted into the instrument insertion channel (16) and led out of the distal end (5) of the endoscope inserting portion (2) in an approximately horizontal direction or an approximately vertical direction of the screen of the endoscope image obtained by the observation means (6).

3. The endoscope (1) according to claim 1, wherein the control mechanism (20) is configured to control the swinging speed at the distal end of the instrument to be fastest on or around a center line equally dividing the approximately horizontal direction or the approximately vertical direction of the screen of the endoscope image which is the same as a swinging direction of the instrument swung by the instrument swing table (10).

4. The endoscope (1) according to any one of claims 1 to 3, wherein the control mechanism includes:
a rotating plate (25) is configured to be rotated by rotational motion at a constant speed from the swing table operating portion (20);
rods (26a, 26b) having one ends thereof rotatably joined to the rotating plate (25) by wheel pins (27a, 27b) and other ends slidably fitted to guide holes (28a, 28b) formed of linear slits; and
brackets (29a, 29b) configured to connect ends of operating wires (32a, 32b) configured to pull the instrument swing table(10) to the other ends of the rods (26a, 26b).

5. The endoscope (1) according to claim 4, wherein, on positioning the instrument led out of the distal end (5) of the endoscope inserting portion (2) on the center line equally dividing the approximately horizontal direction or the approximately vertical direction of the screen of the endoscope image, the rotational position of the rotating plate (25) of the control mechanism (20) is set to maximize a distance between a central axis of the other ends of the rods (26a, 26b) configured to linearly move the brackets (29a, 29b) with the guide holes (28a, 28b) of linear slits and a rotation center of the wheel pins (27a, 27b) configured to rotatably join the one ends of the rods (26a, 26b) to the rotating plate.

6. The endoscope (1) according to claim 4 or 5, wherein the control mechanism (20) includes stopper members (39a, 39b) configured to adjust a rotation range of the rotating plate (25).

## Patentansprüche

1. Endoskop (1), mit:
einer Beobachtungseinrichtung (6), die an einem distalen Ende (5) eines Endoskopeinführbereichs (2) vorgesehen ist;
einem Instrumenteinführungskanal (16), der am Endoskopeinführbereich (2) vorgesehen ist;
einem Instrumentschwenktisch (10), der dazu ausgestaltet ist, ein Instrument zu schwenken, das in den Instrumenteinführkanal (16) eingeführt ist und aus dem distalen Ende des Endoskopeinführbereichs (2) herausgeführt wird; und
einem Schwenktischbetätigungsbereich (19), der dazu ausgestaltet ist, den Instrumentschwenktisch (10) nutzerseitig aus der Ferne zu betätigen,
**gekennzeichnet durch** einen Steuerungsmechanismus (20), der dazu ausgestaltet ist, das Schwenken des Instrumentschwenktisches (10) so zu steuern, dass, wenn der Schwenktischbetätigungsbereich (19) mit einer konstanten Geschwindigkeit arbeitet, eine Schwenkgeschwindigkeit in einem Fall, bei dem das distale Ende des Instrumentes aus dem distalen Ende (5) des Endoskopeinführungsbereichs (2) an einem Mittelpunkt (Q) eines Bildschirms eines Endoskopbildes, das durch die Beobachtungseinrichtung (6) erhalten wird, schneller als die Schwenkgeschwindigkeit in dem Fall ist, bei dem sich das entfernte Ende des Instrumentes in einem peripheren Teil des Bildschirms des Endoskopbildes befindet.

2. Endoskop (1) nach Anspruch 1, wobei der Instrumentschwenktisch (10) dazu ausgebildet ist, das Instrument, das in den Instrumenteinführungskanal (16) eingeführt ist und aus dem distalen Ende (5) des Endoskopeinführungsbereiches (2) herausgeführt wird, in einer näherungsweise horizontalen Richtung oder einer näherungsweise vertikalen Richtung des Bildschirms des Endoskopbildes, das durch die Beobachtungseinrichtung (6) erhalten wird, zu schwenken.

3. Endoskop (1) nach Anspruch 1, wobei der Steuerungsmechanismus (20) dazu ausgebildet ist, die Schwenkgeschwindigkeit an dem distalen Ende des Instrumentes so zu steuern, dass sie am oder um eine Mittellinie am höchsten ist, die die näherungsweise horizontale Richtung oder die näherungsweise vertikale Richtung des Bildschirms des Endoskopbildes teilt und die die gleiche wie eine Schwenkrichtung des Instrumentes ist, das durch den Instrumentschwenktisch (10) geschwenkt wird.

4. Endoskop (1) nach einem der Ansprüche 1 bis 3, wobei der Steuerungsmechanismus umfasst:
eine rotierende Platte (25), die dazu ausgebildet ist, durch eine Drehbewegung mit einer konstanten Drehzahl vom Schwenktischbetätigungsbereich (20) gedreht zu werden;
Stäbe (26a, 26b), deren eine Enden drehbar mit der rotierenden Platte (25) durch Stifte (27a, 27b) verbunden sind und deren andere Enden verschiebbar in Führungsöffnungen (28a, 28b) eingepasst sind, die als Längsschlitze gebildet sind; und
Klammern (29a, 29b), die dazu ausgebildet sind, Enden der Betätigungsdrähte (23a, 23b) zu verbinden, die dazu ausgebildet sind, den Instrumentschwenktisch (10) zu den anderen Enden der Stäbe (26a, 26b) zu ziehen.

5. Endoskop (1) nach Anspruch 4, wobei beim Positionieren des Instrumentes, das aus dem distalen Ende (5) des Endoskopeinführungsbereiches (2) auf der Mittellinie herausgeführt wird, die die näherungsweise horizontale Richtung oder die näherungsweise vertikale Richtung des Bildschirms des Endoskopbildes gleichmäßig teilt, die horizontale Stellung der rotierenden Platte (25) des Steuerungsmechanismus (20) so vorgegeben wird, dass sie einen Abstand zwischen einer Mittelachse des anderen Endes der Stäbe (26a, 26b), die dazu ausgestaltet sind, die Klammern (29a, 29b) mit den Führungsöffnungen (28a, 28b) der Längsschlitze linear zu bewegen, und einem Drehmittelpunkt der Radstifte (27a, 27b) maximiert, der dazu ausgebildet ist, drehbar die einen Enden der Stäbe (26a, 26b) mit der Drehplatte zu verbinden.

6. Endoskop (1) nach einem der Ansprüche 4 oder 5, wobei der Steuerungsmechanismus (20) Anschlagelemente (39a, 39b) aufweist, die dazu ausgestaltet sind, einen Drehbereich der Drehplatte (25) einzustellen.

## Revendications

1. Endoscope (1) comprenant:
un moyen d'observation (6) prévu à une extrémité distale (5) d'une partie d'insertion d'endoscope (2);
un canal d'insertion d'instrument (16) prévu sur la partie d'insertion d'endoscope (2);
une table oscillante d'instrument (10) configurée pour faire osciller un instrument inséré dans le canal d'insertion d'instrument (16) et mené à l'extérieur de l'extrémité distale de la partie d'insertion d'endoscope (2); et
une partie d'opération de table oscillante (19) configurée pour opérer à distance la table oscillante d'instrument (10) sur un côté d'utilisateur;
**caractérisé par** un mécanisme de commande (20) configuré pour commander les oscillations de la table oscillante d'instrument (10) de telle sorte que, lors de l'opération de la partie d'opération de table oscillante (19) à une vitesse constante, une vitesse d'oscillation dans le cas où l'extrémité distale de l'instrument mené à l'extérieur de l'extrémité distale (5) de la partie d'insertion d'endoscope (2) est en un centre (Q) d'un écran d'une image d'endoscope obtenue par le moyen d'observation (6), est plus rapide que la vitesse d'oscillation dans le cas où l'extrémité distale de l'instrument est en une partie périphérique de l'écran de l'image d'endoscope.

2. Endoscope (1) selon la revendication 1, dans lequel la table oscillante d'instrument (10) est configurée pour faire osciller l'instrument inséré dans le canal d'insertion d'instrument (16) et mené à l'extérieur de l'extrémité distale (5) de la partie d'insertion d'endoscope (2) dans un sens approximativement horizontal ou un sens approximativement vertical de l'écran de l'image d'endoscope obtenue par le moyen d'observation (6).

3. Endoscope (1) selon la revendication 1, dans lequel le mécanisme de commande (20) est configuré pour commander la vitesse d'oscillation à l'extrémité distale de l'instrument de façon à ce qu'elle soit plus rapide sur une ou autour d'une ligne centrale divisant de façon égale le sens approximativement horizontal ou le sens approximativement vertical de l'écran de l'image d'endoscope qui est le même qu'un sens d'oscillation de l'instrument fait osciller par la table oscillante d'instrument (10).

4. Endoscope (1) selon l'une quelconque des revendications 1 à 3, dans lequel le mécanisme de commande inclut:
une plaque rotative (25) configurée pour être mise en rotation par un mouvement de rotation à une vitesse constante de la partie d'opération de table oscillante (19);
des tiges (26a, 26b) ayant une extrémité de chacune de celles-ci jointe en rotation à la plaque rotative (25) par des axes de pivot (27a, 27b) et une autre extrémité ajustée de façon coulissante sur des trous de guidage (28a, 28b) constitués de fentes linéaires; et
des plaques de fixation (29a, 29b) configurées pour connecter des extrémités de fils d'opération (32a, 32b) configurés pour tirer la table oscillante d'instrument (10) jusqu'aux autres extrémités des tiges (26a, 26b).

5. Endoscope (1) selon la revendication 4, dans lequel, au positionnement de l'instrument mené à l'extérieur de l'extrémité distale (5) de la partie d'insertion d'endoscope (2) sur la ligne centrale divisant de façon égale le sens approximativement horizontal ou le sens approximativement vertical de l'écran de l'image d'endoscope, la position en rotation de la plaque rotative (25) du mécanisme de commande (20) est fixée de façon à maximiser une distance entre un axe central des autres extrémités des tiges (26a, 26b) configurées pour déplacer linéairement les plaques de fixation (29a, 29b) avec les trous de guidage (28a, 28b) de fentes linéaires et un centre de rotation des axes de pivot (27a, 27b) configurés pour joindre en rotation une extrémité de chacune des tiges (26a, 26b) à la plaque rotative.

6. Endoscope (1) selon la revendication 4 ou 5, dans lequel le mécanisme de commande (20) inclut des éléments d'arrêt (39a, 39b) configurés pour ajuster une plage de rotation de la plaque rotative (25).
